# EUROPEAN PATENT SPECIFICATION

(11) **EP 2 495 039 B1**
(45) Date of publication and mention of the grant of the patent: **19.10.2016**
(21) Application number: 10826853.3
(22) Date of filing: 29.10.2010
(51) Int. Cl.: B01J 19/00, C12M 1/00, B01L 7/00

(54) **TEMPERATURE CONTROLLING UNIT AND TEMPERATURE CONTROLLING METHOD**
TEMPERATURSTEUERUNGSEINHEIT UND TEMPERATURSTEUERUNGSVERFAHREN
UNITÉ DE RÉGULATION THERMIQUE ET PROCÉDÉ DE RÉGULATION THERMIQUE

(30) Priority: 30.10.2009 JP 2009251040
(43) Date of publication of application: 05.09.2012
(73) Proprietor: ARKRAY, Inc., Minami-ku Kyoto-shi Kyoto 601-8045 (JP)
(72) Inventor: KITAMURA Shigeru, Kyoto-shi Kyoto 6020008 (JP); NAKANISHI Naoyuki, Kyoto-shi Kyoto 6020008 (JP)
(74) Representative: Piésold, Alexander James
(86) International application number: PCT/JP2010/069290
(87) International publication number: WO 2011/052723

(56) References cited:
- WO-A2-2008/027398
- DE-B4- 19 646 114
- JP-A- 6 277 036
- JP-A- 8 117 590
- JP-A- 2000 270 837
- JP-A- 2002 306 154
- JP-A- 2006 238 848
- US-B2- 7 030 340

## Description

### TECHNICAL FIELD

The present invention relates to a temperature controlling unit that can be used as a PCR machine, for example. The present invention also relates to a temperature controlling method that can be used for PCR methods.

### BACKGROUND ART

Apparatuses for controlling the temperature of a liquid are currently used in various technical fields. For instance, in biochemistry, temperature controlling units for controlling the temperature of a sample liquid are used. Examples of known temperature controlling units include a PCR machine for performing a PCR (polymerase chain reaction) method. As to PCR methods, description is given in e.g. Patent Documents 1 and 2 identified below.

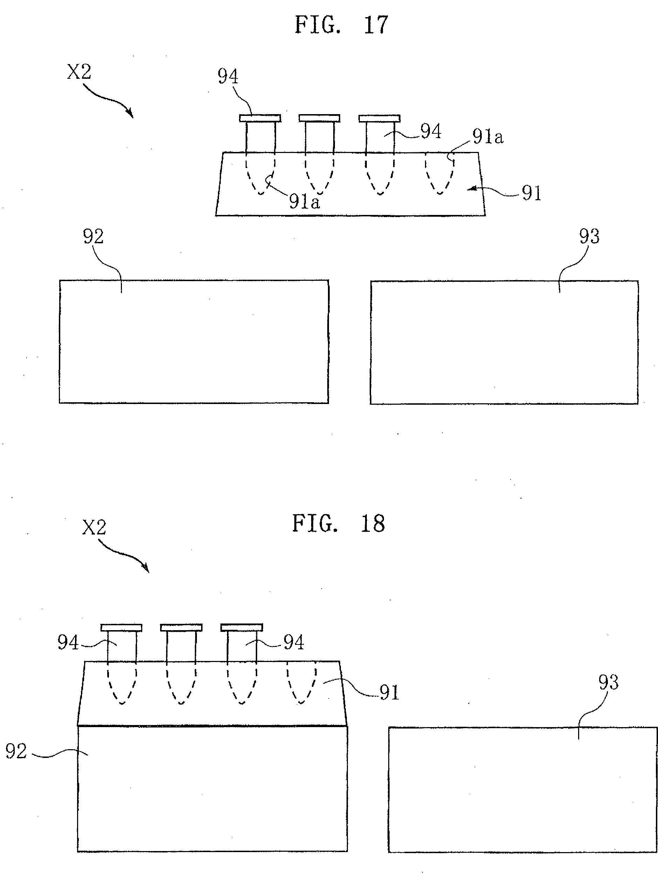

Fig. 17 shows an example of conventional PCR machine. The illustrated PCR machine X2 includes a holding block 91, a heating block 92 and a cooling block 93. In the PCR machine X2, a cycle including thermal denaturation, annealing and elongation is repeated a plurality of times.

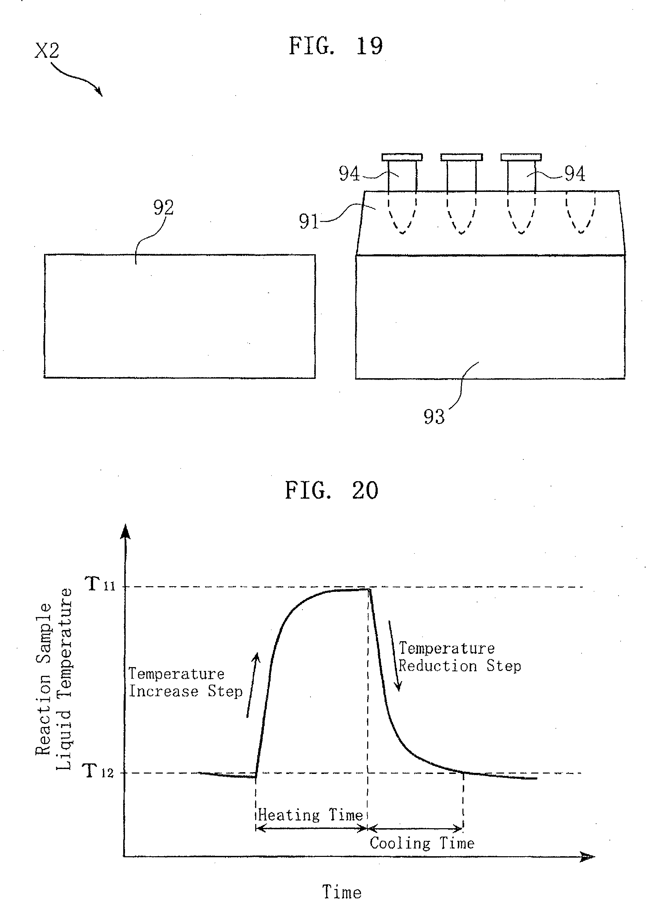

The holding block 91 is formed with a plurality of recesses 91a for receiving tubes 94. Each of the tubes 94 contains a reaction sample liquid or the like for performing a PCR method. The reaction sample liquid contains template DNA, primer DNA, DNA polymerase, and dNTP. The holding block 91 is transferred by a transfer member (not shown) to a position above the heating block 92 (Fig. 18) or a position above the cooling block 93 (Fig. 19). The heating block 92 is provided for supplying heat to the holding block 91 and thermally connected to a heating device (not shown). The cooling block 93 is provided for taking heat from the holding block 91 and thermally connected to a heat-absorbing device (not shown).

In the PCRmachine X2 , a PCRmethod is performed as described below, for example.

First, the holding block 91 is placed on the heating block 92 and heated by the heating block 92 (temperature increase step). In this step, the heating block 92 is kept at a thermal denaturation temperature T₁₁ (e.g. 95 °C) by the heating device.

When the holding block 91 substantially reaches the thermal denaturation temperature T₁₁, the reaction sample liquid in the tubes 94 held by the holding block 91 also reaches the denaturation temperature T₁₁, so that a thermal denaturation step starts. In the thermal denaturation step, two strands of a template DNA are separated from each other.

After the thermal denaturation step, the holding block 91 is transferred to and placed on the cooling block 93 and cooled by the cooing block 93 (temperature reduction step). In this step, the cooling block 93 is kept at an annealing/elongation temperature T₁₂ (e.g. 60 °C) by the operation of the heat-absorbing device, not shown.

When the holding block 91 substantially reaches the annealing/elongation temperature T₁₂, the reaction sample liquid in the tubes 94 held by the holding block 91 also reaches the annealing/elongation temperature T₁₂, so that an annealing/elongation step (the step in which annealing and elongation proceed at the same time) starts. In the annealing step, each single-stranded DNA of the template combines with a primer (containing a base sequence complementary to part of the single-stranded DNA). In the elongation step, at the 3' end of the primer combined with the single-stranded DNA of the template, a DNA strand containing a base sequence complementary to a single-stranded DNA is elongated or synthesized.

In the PCR machine X2, the cycle including the above-described steps is repeated a plurality of times, whereby a piece of DNA having a predetermined base sequence is amplified.
Patent Document 1: JP-A-4-501530
Patent Document 2: JP-A-6-277036

Fig. 20 is a graph showing an example of temperature change of a reaction sample liquid in each cycle of the above-described PCR method performed by the PCR machine X2. As shown in the graph of Fig. 20, in the temperature increase step, the temperature increase speed in a temperature range close to the target temperature (thermal denaturation temperature T₁₁) is considerably lowas compared with the temperature increase speed in the initial stage of the temperature increase step. In this way, with the PCR machine X2, the reaction sample liquid reaches the thermal denaturation temperature T₁₁ after going through the temperature range in which the temperature increase speed is considerably low. Thus, it is necessary to secure sufficient time for the temperature increase step. Moreover, in the temperature reduction step, the temperature reduction speed in a temperature range close to the target temperature (the annealing/elongation temperature T₁₂) is considerably low as compared with the temperature reduction speed in the initial stage of the temperature reduction step. In this way, with the PCR machine X2, the reaction sample liquid reaches the annealing/elongation temperature T₁₂ after going through the temperature range in which the temperature reduction speed is considerably low. Thus, it is necessary to secure sufficient time for the temperature reduction step as well. Thus, the PCR machine X2 is not suitable for completing the temperature increase step and the temperature reduction step in a short period of time. In other words, the PCR machine X2 is not suitable for quickly changing the temperature of a reaction sample liquid (liquid).

Another thermocycler for PCR is described in US 7030340.

### SUMMARY OF THE INVENTION

The present invention has been proposed under the circumstances described above. It is therefore an object of the present invention to provide a temperature controlling unit and a temperature controlling method suitable for quickly changing the temperature of a liquid.

According to a first aspect of the present invention, there is provided a temperature controlling unit comprising:
a holder for holding a liquid receiver containing a liquid in contact with the liquid receiver, the holder being configured to maintain a first temperature (T₁) for keeping temperature of the liquid at a lower target temperature (T_{L});
a heating block for increasing the temperature of the liquid through contact with the liquid receiver, the heating block being movable relative to the liquid receiver and configured to maintain a second temperature (T₂) higher than a higher target temperature (T_{H}) that is higher than the lower target temperature (T_{L}); and
a cooling block for reducing the temperature of the liquid through contact with the liquid receiver, the cooling block being movable relative to the liquid receiver and configured to maintain a third temperature (T₃) lower than the lower target temperature (T_{L});
wherein: the holder includes a holding surface for holding the liquid receiver and is rotatable about an axis perpendicular to the holding surface; and
each of the heating block and the cooling block faces the holding surface of the holder and is movable toward and away from the holding surface.

The liquid or target, subjected to temperature control by the temperature controlling unit, is received in a liquid receiver, and the liquid receiver is held by a holder. During the operation of the unit, the temperature of the holder is set to and maintained at a first temperature for keeping the temperature of the liquid in the liquid receiver at a lower target temperature. Here, the first temperature for keeping the temperature of the liquid in the liquid receiver at a lower target temperature, is a temperature by which the temperature of the liquid in the liquid receiver will be changed and subsequently kept at the lower target temperature when a sufficient time has elapsed in a state where no heat transfer occurs from the heating block to the liquid receiver or the liquid and no heat transfer from the liquid receiver or the liquid to the cooling block. The above-defined first temperature may be set depending on, for example, the lower target temperature, environmental temperature, thermal conductivity of the material for the liquid receiver, and the structure and heat dissipation ability of the liquid receiver. For instance, when the lower target temperature is equal or substantially equal to the environmental temperature, it may be suitable to set the first temperature of the holder to be equal to the lower target temperature. When the lower target temperature is considerably higher than the environmental temperature, it may be suitable to set the first temperature of the holder to be higher than the lower target temperature. When the lower target temperature is considerably lower than the environmental temperature, it may be suitable to set the first temperature of the holder to be lower than the lower target temperature.

The temperature increase by the temperature controlling unit is performed by causing the heating block, which is movable relative to the liquid receiver, to come closer to and into contact with the liquid receiver. At least during the temperature increase step, the temperature of the heating block is set to and maintained at a second temperature. It is preferable that the temperature of the heating block is maintained at the second temperature during the operation of the unit. The second temperature is higher than a higher target temperature (that is higher than the lower target temperature) for the liquid in the liquid receiver. For instance, in the temperature increase step by the temperature control unit, heat transfer from the heating block to the liquid receiver or the liquid is stopped by separating the heating block from the liquid receiver when the temperature of the liquid in the liquid receiver has reached the higher target temperature.

The temperature reduction by the temperature controlling unit is performed by causing the cooling block, which is movable relative to the liquid receiver held by the holder kept at the first temperature, to come closer to and into contact with the liquid receiver. At least during the temperature reduction step, the temperature of the cooling block is set to and maintained at a third temperature. It is preferable that the temperature of the cooling block is maintained at the third temperature during the operation of the unit. The third temperature is lower than the lower target temperature for the liquid in the liquid receiver. When the first temperature of the holder is lower than the lower target temperature, the third temperature of the cooling block is set to be lower than the first temperature. For instance, in the temperature reduction step by the temperature control unit, heat transfer from the liquid receiver or the liquid to the cooling block is stopped by separating the cooling block from the liquid receiver before the temperature of the liquid in the liquid receiver reaches the lower target temperature.

Preferably, in the first aspect of the present invention, the first temperature of the holder may be equal to the lower target temperature; or higher than the lower target temperature and lower than the higher target temperature; or lower than the lower target temperature and higher than the third temperature. The first temperature of the holder may be set depending on the lower target temperature, environmental temperature, thermal conductivity of the material for the liquid receiver, and the structure and heat dissipation ability of the receiver, such that the temperature of the liquid in the liquid receiver is ultimately kept at the lower target temperature when a sufficient time has elapsed in a state where no heat transfer occurs from the heating block to the liquid receiver or the liquid and no heat transfer from the liquid receiver or the liquid to the cooling block.

Preferably, the heating block is configured to come into contact with a side of the liquid receiver that is opposite from the holder, and the cooling block is configured to come into contact with a side of the liquid receiver that is opposite from the holder.

Preferably, the holding surface includes a first region for holding a liquid receiver containing a liquid in contact with the liquid receiver and a second region for holding a liquid receiver containing a liquid in contact with the liquid receiver. In this case, each of the heating block and the cooling block is configured to move closer to and come into contact with the liquid receiver held in the first region when facing the first region and configured to move closer to and come into contact with the liquid receiver held in the second region when facing the second region.

Preferably, the holding surface includes a first region for holding a plurality of liquid receivers each containing a liquid in contact with the liquid receivers and a second region for holding a plurality of liquid receivers each containing a liquid in contact with the liquid receivers. In this case, each of the heating block and the cooling block is configured to move closer to and come into contact with the plurality of liquid receivers held in the first region when facing the first region and configured to move closer to and come into contact with the plurality of liquid receivers held in the second region when facing the second region.

Preferably, the first region and the second region are configured to hold the plurality of liquid receivers such that the liquid receivers are arranged on a circle (imaginary circle) around the axis.

Preferably, the liquid receiver includes a first cell wall and a second cell wall facing and spaced from each other, and a cell for receiving a liquid defined between the first cell wall and the second cell wall. In this case, the holder is configured to hold the liquid receiver in contact with the first cell wall of the liquid receiver. The heating block is configured to come into contact with the second cell wall of the liquid receiver, and the cooling block is also configured to come into contact with the second cell wall of the liquid receiver.

Preferably, the maximum dimension of the cell in a direction perpendicular to the spacing direction in which the first cell and the second cell are spaced from each other is larger than the maximum dimension of the cell in the spacing direction. That is, it is preferable that the cell for receiving a liquid as the target for temperature control is shallow.

Preferably, each of the heating block and the cooling block includes a projection for coming into contact with the second cell wall. The heating block with a projection for coming into contact with the second cell wall is suitable for allowing local heat transfer from the heating block to the liquid in the cell. The cooling block with a projection for coming into contact with the second cell wall is suitable for allowing local heat transfer from the liquid in the cell to the cooling block. Realizing local heat transfer contributes to enhancement of heat transfer efficiency.

According to a second aspect of the present invention, there is provided a temperature controlling method for controlling the temperature of a liquid by using the controlling unit according to the first aspect of the invention, the method comprising:
providing a liquid in the liquid receiver held in the holder;
a temperature increase step for bringing the heating block kept at a heating temperature (T₂) (corresponding to the second temperature in the first aspect) higher than a higher target temperature (T_{H}) for the liquid into contact with the liquid receiver containing the liquid to increase the temperature of the liquid; and
a temperature reduction step for bringing the cooling block kept at a cooling temperature (T₃) (corresponding to the third temperature in the first aspect) lower than a lower target temperature (T_{L}) that is lower than the higher target temperature (T_{H}) into contact with the liquid receiver to reduce the temperature of the liquid;
wherein the holder is maintained at the first temperature (T₁) for keeping a temperature of the liquid at the lower target temperature (T_{L}), said first temperature (T₁) being any one of a temperature equal to the lower target temperature (T_{L}), a temperature higher than the lower target temperature (T₁) and lower than the higher target temperature (T_{H}), and a temperature lower than the lower target temperature (T_{L}) and higher than the cooling temperature (T₃) (the temperature of the lower target temperature maintaining member corresponds to the first temperature in the first aspect); and
each of the heating block and the cooling block is movable toward and away from the holder.

The temperature controlling method can be carried out properly by the above-described temperature controlling unit according to the first aspect. The temperature controlling method is suitable for quickly changing (increasing or reducing) the temperature of a liquid and also suitable for controlling the temperature of a liquid precisely to a higher target temperature or a lower target temperature. The temperature controlling method is suitable for the application to e.g. a PCR method that requires quick and precise temperature control.

Preferably, in the second aspect of the present invention, the heating block is separated from the liquid receiver in the temperature increase step when the temperature of the liquid has reached the higher target temperature. This is suitable for controlling the temperature of a liquid during the
temperature increase precisely to the higher target temperature in the temperature increase step.

Preferably, in the temperature reduction step, the cooling block is separated from the liquid receiver before the temperature of the liquid reaches the lower target temperature. This contributes to controlling the temperature of a liquid during the temperature reduction precisely to the lower target temperature in the temperature reduction step.

### BRIEF DESCRIPTION OF THE DRAWINGS

Fig. 1 shows part of the structure of a temperature controlling unit according to the present invention;
Fig. 2 shows part of a functional block diagram of the temperature controlling unit according to the present invention;
Fig. 3 is a view seen in the direction of arrow III-III in Fig. 1, showing a holding surface of a rotation table, with sample liquid chips held thereon;
Fig. 4 is a view seen in the direction of arrow IV-IV in Fig. 1, showing a surface of a heating block on the rotation table side and a surface of the cooling block on the rotation table side;
Fig. 5A is an enlarged plan view of a sample liquid chip;
Fig. 5B is a sectional view taken along lines V-V in Fig. 5A;
Fig. 6A shows a process in introducing sample liquid into a sample liquid chip;
Fig. 6B shows a process in introducing sample liquid into a sample liquid chip;
Fig. 6C shows a process in introducing sample liquid into a sample liquid chip;
Fig. 7 shows part of a table of steps inparallel temperature control performed by the temperature controlling unit according to the present invention;
Fig. 8 shows the state of the temperature controlling unit in Steps 1 and 6;
Fig. 9 shows the state of the temperature controlling unit in Step 2;
Fig. 10 shows the state of the temperature controlling unit in Step 3;
Fig. 11 shows the state of the temperature controlling unit in Step 4;
Fig. 12 shows the state of the temperature controlling unit in Step 5;
Fig. 13 is an enlarged sectional view showing part of the temperature controlling unit during a temperature increase step;
Fig. 14 is an enlarged sectional view showing part of the temperature controlling unit during a temperature reduction step;
Fig. 15 is a graph showing part of temperature change of a sample liquid in an Example;
Fig. 16 is a graph showing part of temperature change of a sample liquid in a Comparative Example;
Fig. 17 shows the structure of a conventional PCR machine;
Fig. 18 shows the PCRmachine of Fig. 17 during a temperature increase step;
Fig. 19 shows the PCRmachine of Fig. 17 during a temperature reduction step; and
Fig. 20 is a graph showing an example of temperature change of a reaction sample liquid in each cycle of the PCR method performed by the PCR machine of Fig. 17.

### BEST MODE FOR CARRYING OUT THE INVENTION

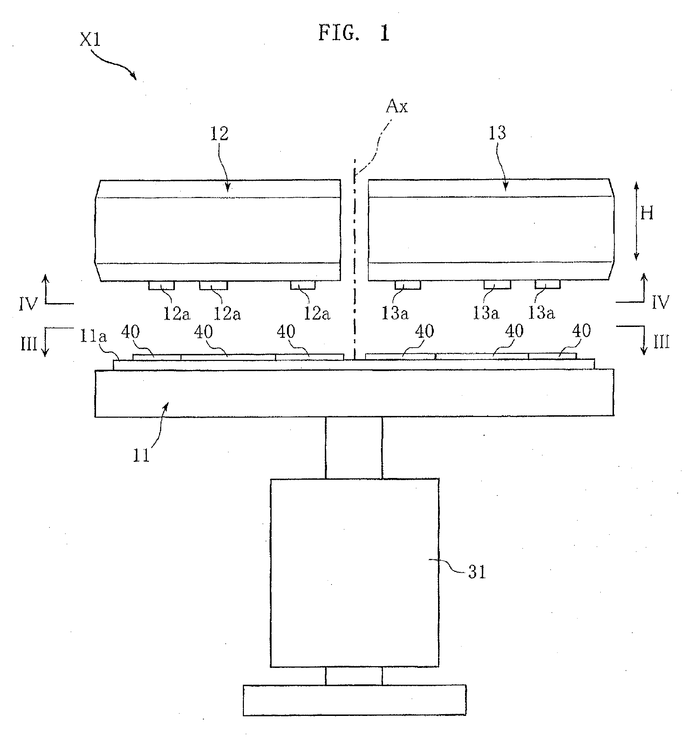

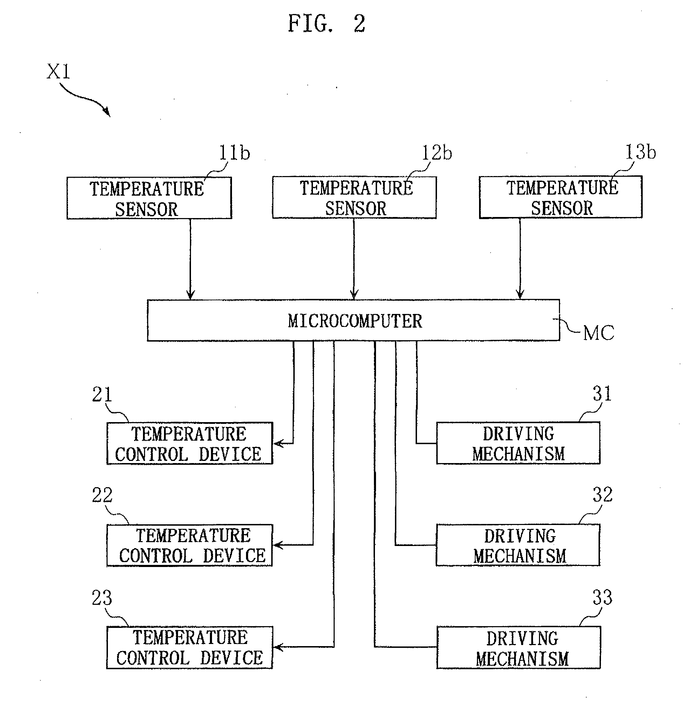

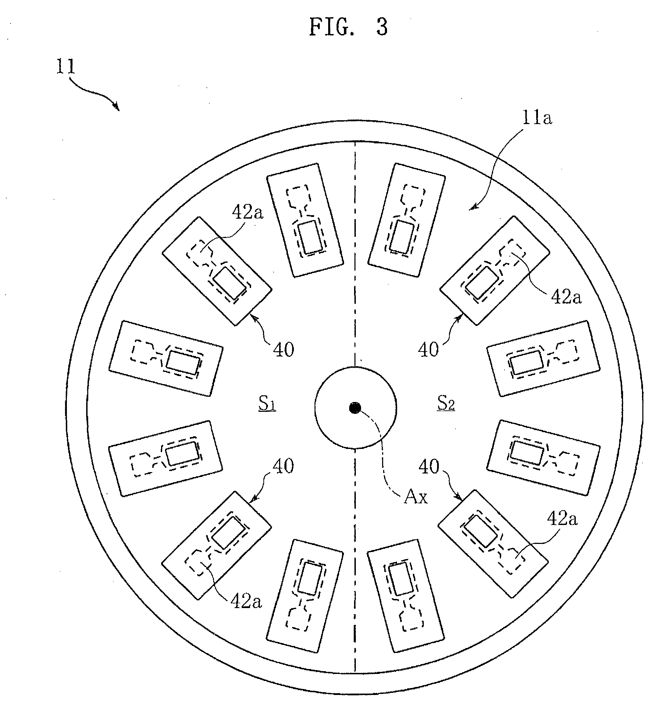

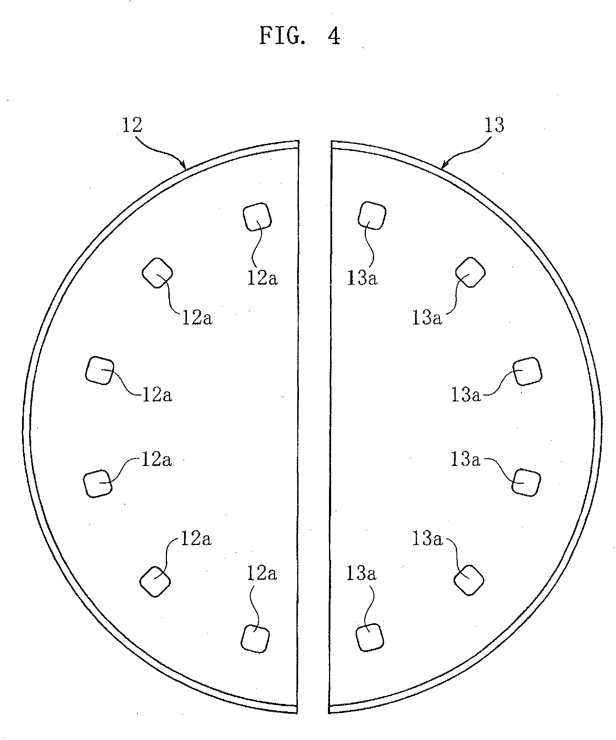

A temperature controlling unit X1 according to the present invention is shown in Figs. 1-4. Fig. 1 shows part of the structure of the temperature controlling unit X1. Fig. 2 shows part of a functional block diagram of the temperature controlling unit X1. Figs. 3 and 4 are views seen in the direction of arrows III-III and arrows IV-IV in Fig. 1, respectively.

The temperature controlling unit X1 includes a rotation table 11, a heating block 12, a cooling block 13, temperature controlling devices 21, 22, 23, driving mechanisms 31, 32, 33 and a microcomputer MC. The temperature controlling unit X1 is designed to perform a PCRmethod that repeats a cycle including thermal denaturation, annealing and elongation a plurality of times.

The rotation table 11 functions as a holder and a lower target temperature maintaining member. The rotation table 11 includes a holding surface 11a for holding sample liquid chips 40 in contact with the sample liquid chips 40. The rotation table 11 is rotatable about an axis Ax (perpendicular to the holding surf ace 11a) shown in Figs. 1 and 3. The holding surf ace 11a includes a first region S₁ and a second region S₂ each of which includes a plurality of sample liquid chip mount portions. (For clarity, the boundary between the first region S₁ and the second region S₂ is indicated by a phantom line in Fig. 3.) In this embodiment, the maximum number of sample liquid chips 40 that can be held in the first region S₁ and the maximum number of sample liquid chips 40 that can be held in the second region S₂ are equal to each other. In this embodiment, the sample liquid chips 40 are held on the holding surface 11a as arranged on an imaginary circle around the axis Ax.

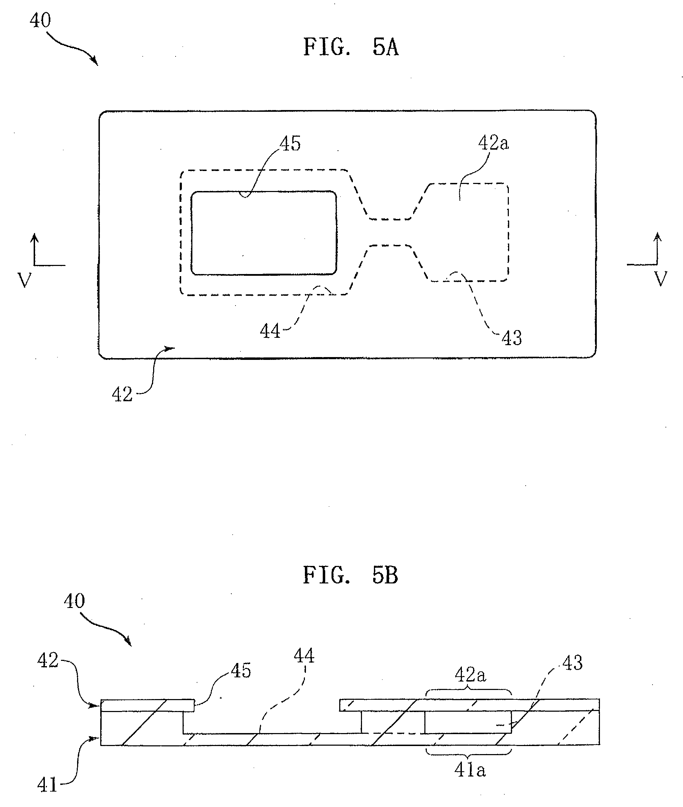

The specific structure of each sample liquid chip 40 is shown in Figs. 5A and 5B. Fig. 5A is an enlarged plan view of the sample liquid chip 40. Fig. 5B is a sectional view taken along lines V-V in Fig. 5A. The sample liquid chip 40 is provided by bonding a main body 41 having a recess and a cover 42 having an opening. The sample liquid chip 40 includes a sample liquid cell 43 defined between cell walls 41a and 42a facing and spaced from each other, a liquid retaining space 44 communicating with the sample liquid cell 43, and an introduction port 45 provided at a position corresponding to the liquid retaining space 44. The main body 41 and the cover 42 can be made by resin molding. Examples of resin material for making the main body 41 and the cover 42 include PS, PC, PMMA, COC and COP. The cell wall 41 a is part of the main body 41, whereas the cell wall 42a is part of the cover 42. The thickness of the cell wall 41 a, 42a (the thickness shown in Fig. 5B) is e.g. 10 to 500 µm. The sample liquid cell 43 is a space for receiving a predetermined sample liquid or the like for performing the PCR method (not shown in Figs. 5A and 5B). The sample liquid cell 43 is shallow. Specifically, the maximum dimension of the sample liquid cell 43 in a direction perpendicular to the spacing direction of the cell walls 41 a and 42a (e.g. 1000 µm) is larger than the maximum dimension of the sample liquid cell 43 in the spacing direction (e.g. 500 µm). The volume of the sample liquid cell 43 is e.g. 0.1 to 100 µL. A sample liquid containing template DNA, primer DNA, DNA polymerase, and dNTP is introduced into the sample liquid cell 43. The liquid retaining space 44 is a space for preparing the sample liquid to be introduced into the sample liquid cell 43 by mixing various kinds of reagents or the like. The introduction port 45 is used for supplying various kinds of reagents or the like into the liquid retaining space 44.

As shown in Fig. 3, in mounting a sample liquid chip 40 onto the holding surface 11 a (which is rotatable), the sample liquid chip 40 is arranged in a sample liquid chip mount portion such that the sample liquid cell 43 is positioned on a radially outer side of the holding surface 11a and the liquid retaining space 44 is positioned on a radially inner side of the holding surface 11 a. The sample liquid chip 40 is removably mounted to the holding surface 11a of the rotation table 11. Specifically, for instance, a plurality of recesses (not shown) may be formed on a side of the sample liquid chip 40 that is to come into contact with the holding surface 11 a (i.e., the
main body 41 side), whereas a plurality of projections (not shown) for fitting into the recesses may be formed on the holding surface 11a in each of the sample liquid chip mount portions at locations corresponding to the recesses. Further, a clipping mechanism for clipping the sample liquid chip 40 onto the holding surface 11a, with the above-described projections fitted in the above-described recesses, may be provided at each sample liquid chip mount portion. By employing this structure in the temperature controlling unit X1, each of the sample liquid chips 40 can be removably held at a predetermined position in the holding surface 11a of the rotation table 11. When the sample liquid chips 40 are held on the holding surface 11a of the rotation table 11, the holding surface 11a comes into contact with the main body 41 side (including the cell wall 41a) of each sample liquid chip 40.

A temperature sensor 11b for detecting the temperature of the holding surface 11a is provided on the holding surface 11a or inside the rotation table 11 adjacent to the holding surface 11a. For instance, the temperature sensor 11b comprises a thermistor. As shown in Fig. 2, the temperature sensor 11b is connected to the microcomputer MC. Signals outputted from the temperature sensor 11b are inputted into the microcomputer MC.

The temperature control device 21 (not shown in Fig. 1) is arranged in the rotation table 11. The temperature control device 21 is thermally connected to the holding surface 11a of the rotation table 11. The temperature control device 21 comprises a Peltier module that utilizes Peltier effect. As shown in Fig. 2, the temperature control device 21 is connected to the microcomputer MC. The amount and direction of electric current to be applied to the Peltier module (temperature control device 21) is changed as required in accordance with the instructions from the microcomputer MC. By the operation of the temperature control device 21, the rotation table 11 or at least the holding surface 11a of the rotation table is kept at a first temperature T₁. The first temperature T₁ is a temperature for keeping the sample liquid in the sample liquid chips 40 on the holding surface 11a at a lower target temperature T_{L}. The first temperature T₁ is set appropriately depending on the lower target temperature T_{L} for the sample liquid as a target for temperature control, environmental temperature, thermal conductivity of the material for the sample liquid chips 40 and the structure and heat dissipation ability of the sample liquid chips 40, for example. For instance, when the lower target temperature T_{L} is equal or substantially equal to the environmental temperature, it may be suitable to set the first temperature T₁ to be equal to the lower target temperature T_{L}. For instance, when the lower target temperature T_{L} is considerably higher than the environmental temperature, it may be suitable to set the first temperature T₁ to be higher than the lower target temperature T_{L}. For instance, when the lower target temperature T_{L} is considerably lower than the environmental temperature, it may be suitable to set the first temperature T₁ to be lower than the lower target temperature T_{L}.

The driving mechanism 31 drives the rotation table 11 for rotation. The driving mechanism 31 is connected to the microcomputer MC and operates in accordance with the instructions from the microcomputer MC. The driving mechanism 31 outputs the amount of rotation of the rotation table 11 to the microcomputer MC. The rotation table 11 is fixed to the rotation shaft of the driving mechanism 31.

The heating block 12 is designed to come into contact with the sample liquid chips 40 to heat the sample liquid in the sample liquid cells 43 of the sample liquid chips 40. The heating block 12 is movable relative to the sample liquid chips 40 on the holding surface 11a. Specifically, the heating block 12 faces the holding surface 11a of the rotation table 11 and is movable toward and away from the holding surface 11a or the sample liquid chips 40 on the holding surface 11a in the arrow H direction shown in Fig. 1. The heating block 12 is kept at a second temperature T₂ higher than a higher target temperature T_{H} (that is higher than the above-described lower target temperature T_{L}) for the sample liquid as a target for temperature control. As shown in Figs. 1 and 4, the heating block 12 has a plurality of projections 12a. Each of the projections 12a is arranged to come into contact with the cell wall 42a of the sample liquid chip 40 on the holding surface 11a (i.e., the side of the sample liquid chip 40 opposite from the rotation table 11).

A temperature sensor 12b for detecting the temperature of the heating block 12 is provided in the heating block 12. For instance, the temperature sensor 12b comprises a thermistor. As shown in Fig. 2, the temperature sensor 12b is connected to the microcomputer MC. Signals outputted from the temperature sensor 12b are inputted into the microcomputer MC.

The temperature control device 22 (not shown in Fig. 1) is thermally connected to the heating block 12. The temperature control device 22 is a heater comprising a heating device. The amount of electric current to be applied to the temperature control device 22 is changed as required in accordance with the instructions from the microcomputer MC, whereby the temperature of the temperature control device 22 changes. By the operation of the temperature control device 22, the heating block 12 is kept at the second temperature T₂.

The driving mechanism 32 (not shown in Fig. 1) drives the heating block 12 for translation in the arrow H direction shown in Fig. 1. As shown in Fig. 2, the driving mechanism 32 is connected to the microcomputer MC. The driving mechanism 32 operates in accordance with the instructions from the microcomputer MC and outputs the amount of translation of the heating block 12 to the microcomputer MC. By the operation of the driving mechanism 32, the heating block 12 moves toward and away from the holding surface 11a of the rotation table 11.

The cooling block 13 is designed to come into contact with the sample liquid chips 40 to cool the sample liquid in the sample liquid cells 43 of the sample liquid chips 40. The cooling block 13 is movable relative to the sample liquid chips 40 on the holding surface 11a. Specifically, the cooling block 13 faces the holding surface 11a of the rotation table 11 and is movable toward and away from the holding surface 11a or the sample liquid chips 40 on the holding surface 11a. The cooling block 13 is kept at a third temperature T₃ lower than the lower target temperature T_{L} for the sample liquid as a target for temperature control. The third temperature T₃, which is lower than the lower target temperature T_{L}, is lower than the first temperature T₁ as well. As shown in Figs. 1 and 4, the cooling block 13 has a plurality of projections 13a. Each of the projections 13a is arranged to come into contact with the cell wall 42a of the sample liquid chip 40 on the holding surface 11a (i.e., the side of the sample liquid chip 40 opposite from the rotation table 11).

A temperature sensor 13b for detecting the temperature of the cooling block 13 is provided in the cooling block 13. For instance, the temperature sensor 13b comprises a thermistor. As shown in Fig. 2, the temperature sensor 13b is connected to the microcomputer MC. Signals outputted from the temperature sensor 13b are inputted into the microcomputer MC.

The temperature control device 23 (not shown in Fig. 1) is thermally connected to the cooling block 13. The temperature control device 23 comprises a Peltier module that utilizes Peltier effect. As shown in Fig. 2, the temperature control device 23 is connected to the microcomputer MC. The amount and direction of electric current to be applied to the Peltier module (temperature control device 23) is changed as required in accordance with the instructions from the microcomputer MC. By the operation of the temperature control device 23 , the cooling block 13 is kept at the third temperature T₃.

The driving mechanism 33 (not shown in Fig. 1) drives the cooling block 13 for translation in the arrow H direction shown in Fig. 1. As shown in Fig. 2, the driving mechanism 33 is connected to the microcomputer MC. The driving mechanism 33 operates in accordance with the instructions from the microcomputer MC and outputs the amount of translation of the cooling block 13 to the microcomputer MC. By the operation of the driving mechanism 33, the cooling block 13 moves toward and away from the holding surface 11a of the rotation table 11.

To perform the PCR method in the temperature controlling unit X1, sample liquid chips 40 are mounted on the holding surface 11a of the rotation table 11 and then sample liquid is introduced into the sample liquid chips 40 in the following manner, for example.

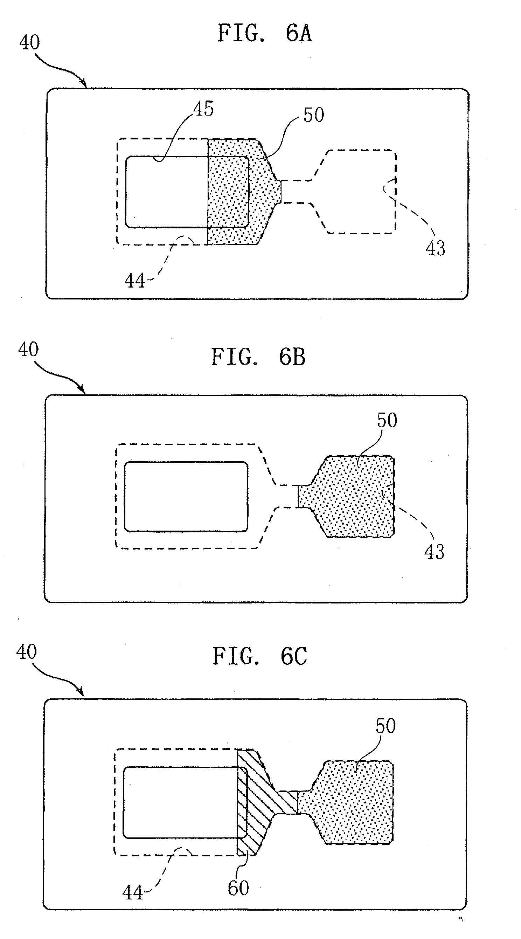

First, as shown in Fig. 3, for example, a necessary number of sample liquid chips 40 are set on the sample liquid chip mount portions in the holding surface 11a of the rotation table 11. (As described above, in this process, each of the sample liquid chips 40 is arranged such that the sample liquid cell 43 is positioned on a radially outer side of the holding surface 11a and the liquid retaining space 44 is positioned on a radially inner side of the holding surface.) The position of each of the sample liquid chips 40 on the holding surface 11a is fixed during the subsequent steps. Then, as shown in Fig. 6A, necessary reagents or the like are introduced into the liquid retaining space 44 through the introduction port 45. Specifically, for example, each reagent may be prepared in the form of a solution and supplied into the liquid retaining space 44. Alternatively, part of the reagents may be prepared in the form of a dried reagent and applied in advance to the bottom surface of the liquid retaining space 44. In this case, other reagents each prepared as a solution are then supplied into the liquid retaining space 44 so that the dried reagent dissolves into the reagents in the form of a solution. Examples of necessary reagents or the like include template DNA, primer DNA, DNA polymerase, dNTP and a buffer component. The reagents are mixed within the liquid retaining space 44 by e.g. pipetting, whereby a sample liquid 50 as a homogenous reaction liquid is obtained. Then, the rotation table 11 is rotated about the axis Ax at a predetermined speed. The centrifugal force acting on the sample liquid 50 due to the rotation of the rotation table 11 causes the sample liquid 50 to move into the sample liquid cell 43, as shown in Fig. 6B. Then, as shown in Fig. 6C, mineral oil 60 is supplied into the liquid retaining space 44. The presence of mineral oil 60 prevents the sample liquid 50 frombeing lost by evaporation, for example, in the subsequent temperature change process.

Then, the rotation table 11 is fixed at a predetermined rotational position about the axis Ax. Specifically, by the operation of the driving mechanism 31, the position of the rotation table 11 is fixed such that the first region S₁ of the holding surface 11a faces the heating block 12 whereas the second region S₂ of the holding surface 11a faces the cooling block 13.

Then, each of the rotation table 11, the heating block 12 and the cooling block 13 is set to a desired temperature and kept at the desired temperature. Specifically, this process is performed in the following manner. The temperature of the rotation table 11 at least at the holding surface 11a is adjusted to the above-described first temperature T₁ by the operation of the temperature control device 21, and the first temperature T₁ is maintained. The temperature of the heating block 12 is adjusted to the above-described second temperature T₂ (heating temperature) by the operation of the temperature control device 22, and the second temperature T₂ is maintained. The temperature of the cooling block 13 is adjusted to the above-described third temperature T₃ (cooling temperature) by the operation of the temperature control device 23, and the third temperature T₃ is maintained. The lower target temperature which the sample liquid 50 should reach in the PCR process is expressed as T_{L} (e.g. 60 °C), whereas the higher target temperature which the sample liquid should reach in the PCR process is expressed as T_{H} (e.g. 95 °C). In such a case, the first temperature T₁ is a temperature by which the temperature of the sample liquid 50 canbe kept at the lower target temperature T_{L} when a sufficient time has elapsed in a state where no heat transfer occurs from the heating block 12 to the sample liquid 50 and no heat transfer from the sample liquid 50 to the cooling block 13. Specifically, the first temperature T₁ may be a temperature equal to the lower target temperature T_{L}, or a temperature higher than the lower target temperature T_{L} and lower than the higher target temperature T_{H}, or a temperature lower than the lower target temperature T_{L} and higher than the third temperature T₃ (cooling temperature). The second temperature T₂ is a temperature higher than the higher target temperature T_{H}. The third temperature T₃ is a temperature lower than the lower target temperature T_{L}.

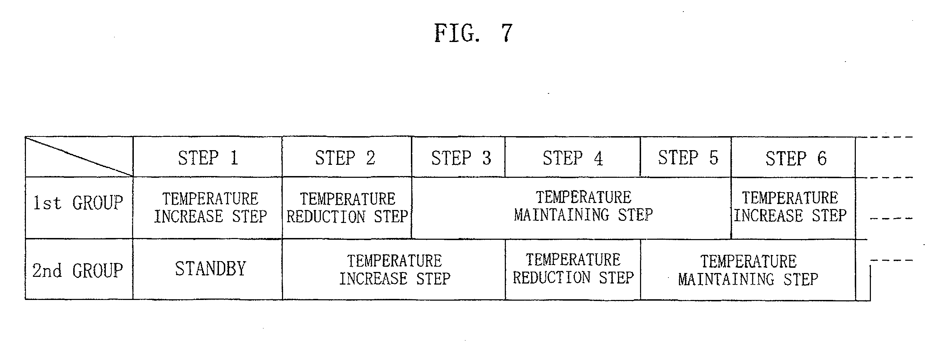

In the temperature controlling unit X1, after the preparation as described above is completed and the temperature of the sample liquid 50 has reached the lower target temperature T_{L}, the PCR method or temperature control is performed in a parallel manner. In this parallel PCR method, the temperature increase step, the temperature reduction step and the temperature maintaining step are performed with respect to the sample liquid 50 in the sample liquid chips 40 held in the first region S₁ (constituting the first group) of the holding surface 11a, while at the same time, the temperature increase step, the temperature reduction step and the temperature maintaining
step are performed with respect to the sample liquid 50 in the sample liquid chips 40 held in the second region S₂ (constituting the second group) of the holding surface 11a. Fig. 7 shows part of a table of steps in the temperature control performed by the temperature controlling unit X1.

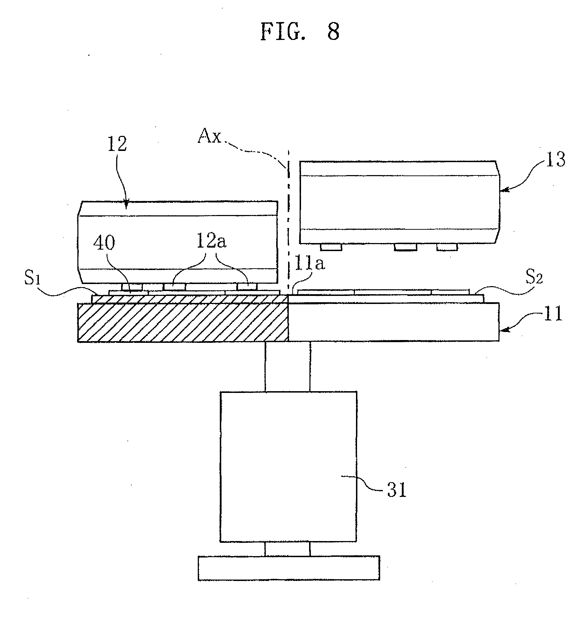

First, in the parallel PCR method by the temperature controlling unit X1, the temperature increase step is performed in Step 1 with respect to the sample liquid chips 40 held in the first region S₁, as shown in Fig. 8. (For clarity, the first region S₁ side of the rotation table 11 is hatched in Fig. 8 and Figs. 9-12 as well.)

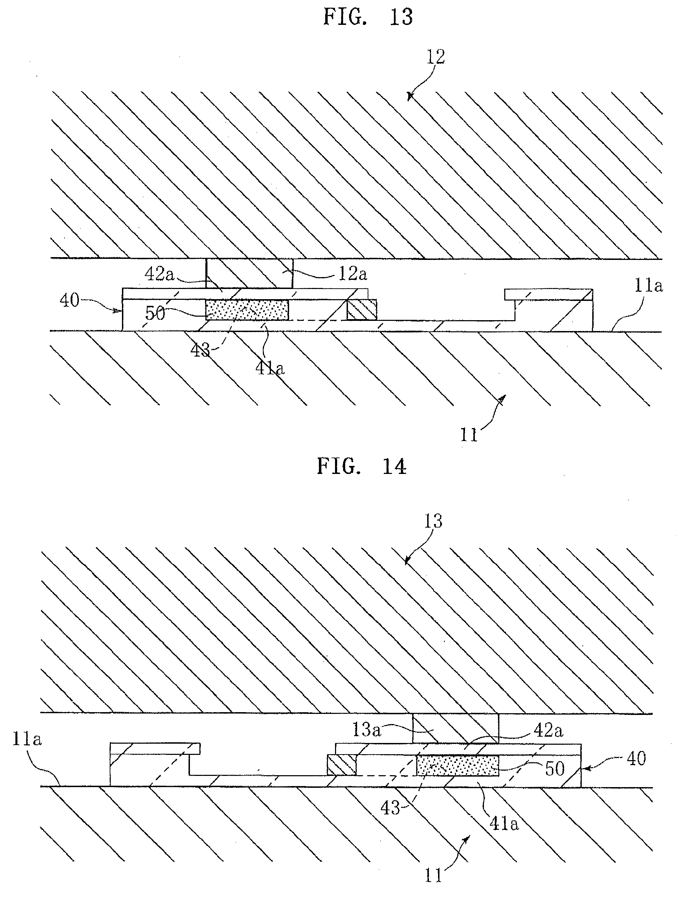

Specifically, in Step 1, the heating block 12 is moved closer to the rotation table 11 to come into contact with the sample liquid chips 40 in the first region S₁ of the holding surface 11a by the operation of the driving mechanism 32, as shown in Fig. 8. More specifically, as shown in Fig. 13, each projection 12a of the heating block 12 is brought into contact with the cell wall 42a of the corresponding sample liquid chip 40. (The cell wall 42a, along with the cell wall 41a, defines the sample liquid cell 43.) By this contact, the temperature increase step with respect to the sample liquid chips 40 of the first group is started. In this temperature increase step, the cell wall 42a of the sample liquid chip 40 is directly heated by the heating block 12 or the projection 12a. By heating the cell wall 42a, heat transfers from the heating block 12 or the projection 12a to the cell wall 42a and the sample liquid 50 in the sample liquid cell 43. Thus, the temperature of the sample liquid 50 increases and reaches the higher target temperature T_{H}. As a result, the two strands of a template DNA in the sample liquid 50 are sufficiently separated from each other (the thermal denaturation step of the first group). When the sample liquid 50 reaches the higher target temperature T_{H}, the heating block 12 or the projection 12a is separated from the cell wall 42a of the sample liquid chip 40. Thus, heat transfer from the heating block 12 to the sample liquid 50 stops (end of the temperature increase step of the first group).

In Step 1, on the other hand, the sample liquid 50 in the sample liquid chips 40 in the second region S₂ (the second group) are kept at a constant temperature (lower target temperature T_{L}) and in a standby state. Any reaction related to PCR does not occur in the sample liquid 50 in these sample liquid chips 40 of the second group.

When Step 1 is finished, the heating block 12 is separated from the sample liquid chips 40 of the first group as described above, and at the same time, the rotation table 11 is rotated 180° about the axis Ax by the operation of the driving mechanism 31. Due to this rotation, the position of the sample liquid chips 40 in the first region S₁ that belong to the first group switches with the position of the sample liquid chips 40 in the second region S₂ that belong to the second group.

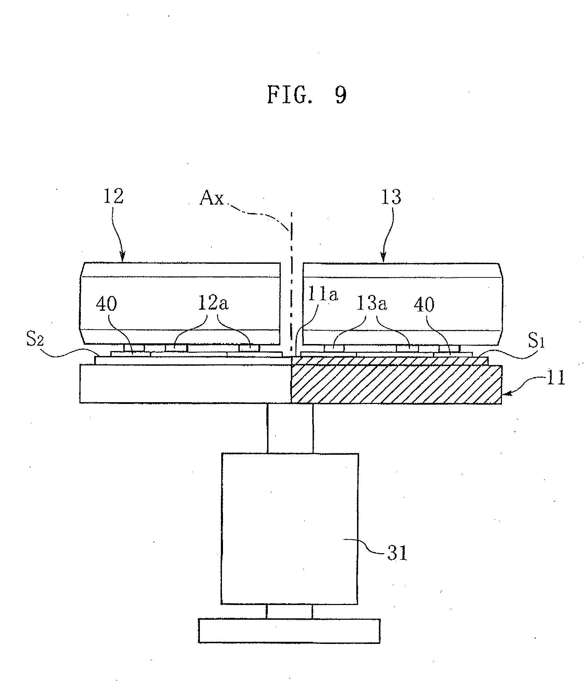

Next, in Step 2, the temperature reduction step is performed with respect to first group, whereas the temperature increase step is performed with respect to the second group, as shown in Fig. 9.

Specifically, in Step 2, the cooling block 13 is moved closer to the rotation table 11 to come into contact with the sample liquid chips 40 in the first region S₁ of the holding surface 11a by the operation of the driving mechanism 33, as shown in Fig. 9. More specifically, as shown in Fig. 14, each projection 13a of the cooling block 13 is brought into contact with the cell wall 42a of the corresponding sample liquid chip 40. (The cell wall 42a, along with the cell wall 41a, defines the sample liquid cell 43.) By this contact, the temperature reduction step with respect to the sample liquid chips 40 of the first group is started. In this temperature reduction step, the cell wall 42a of the sample liquid chip 40 is directly cooled by the cooling block 13 or the projection 13a. Heat transfers from the cell wall 42a and the sample liquid 50 in the sample liquid cell 43 to the cooling block 13 or the projection 13a. Thus, the temperature of the sample liquid 50 reduces. During the temperature reduction, annealing gradually proceeds within the sample liquid 50 (part of the annealing step of the first group). In this annealing step, each single-stranded DNA of the template combines with a primer (containing a base sequence complementary to part of the single-stranded DNA). The cooling block 13 or the projection 13a is separated from the cell wall 42a of the sample liquid chip 40 by the operation of the driving mechanism 33 immediately before(e.g. 10 to 1000 milliseconds before) the sample liquid 50 reaches the lower target temperature T_{L}. Thus, heat transfer to the cooling block 13 stops (end of the temperature reduction step of the first group; end of Step 2) .

In Step 2 , on the other hand, the heating block 12 is moved closer to the rotation table 11 to come into contact with the sample liquid chips 40 in the second region S₂ of the holding surface 11a by the operation of the driving mechanism 32, as shown in Fig. 9. More specifically, as shown in Fig. 13, each projection 12a of the heating block 12 is brought into contact with the cell wall 42a of the corresponding sample liquid chip 40. By this contact, the temperature increase step with respect to the sample liquid chips 40 of the second group is started. In this temperature increase step, the cell wall 42a of the sample liquid chip 40 is directly heated by the heating block 12 or the projection 12a. Heat transfers from the heating block 12 or the projection 12a to the cell wall 42a and the sample liquid 50 in the sample liquid cell 43. Thus, the temperature of the sample liquid 50 increases.

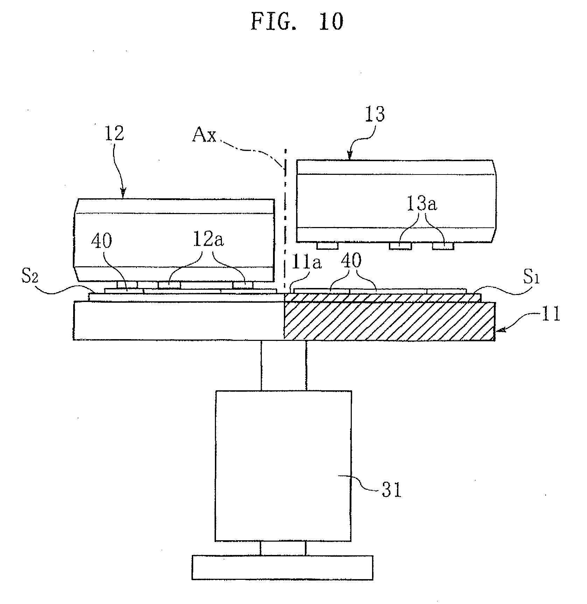

Next, in Step 3, the temperature maintaining step is performed with respect to the first group, whereas the temperature increase step is performed continuously from Step 2 with respect to the second group, as shown in Fig. 10.

In Step 3, the sample liquid chips 40 of the first group are left in contact with the holding surface 11a and the sample liquid 50 in each of the sample liquid chips 40 is kept at a constant temperature (the lower target temperature T_{L}) (the temperature maintaining step of the first group). In the sample liquid 50 in this state, annealing (part of the annealing step of the first group) and elongation (part of the elongation step of the first group) proceed at the same time. In the annealing step, as described above, each single-stranded DNA of the template combines with a primer (containing a base sequence complementary to part of the single-stranded DNA). In the elongation step, at the 3' end of the primer combined with the single-stranded DNA of the template, a DNA strand containing base sequence complementary to single-stranded DNA is elongated or synthesized.

In Step 3, continuously from Step 2, the cell wall 42a of each sample liquid chip 40 of the second group is directly heated by the heating block 12 or the projection 12a, and heat transfers from the heating block 12 or the projection 12a to the cell wall 42a and the sample liquid 50 in the sample liquid cell 43. When the sample liquid 50 reaches the higher target temperature T_{H}, the two strands of a template DNA in the sample liquid 50 are sufficiently separated from each other (thermal denaturation step of the second group). When the sample liquid 50 reaches the higher target temperature T_{H}, the heating block 12 or the projection 12a is separated from the cell wall 42a of the sample liquid chip 40 by the operation of the driving mechanism 32. Thus, heat transfer from the heating block 12 to the sample liquid 50 stops (end of the temperature increase step of the first group).

When Step 3 is finished, the heating block 12 is separated from the sample liquid chips 40 of the second group as described above, and at the same time, the rotation table 11 is rotated 180° about the axis Ax by the operation of the driving mechanism 31. Due to this rotation, the position of the sample liquid chips 40 in the first region S₁ that belong to the first group switches with the position of the sample liquid chips 40 in the second region S₂ that belong to the second group.

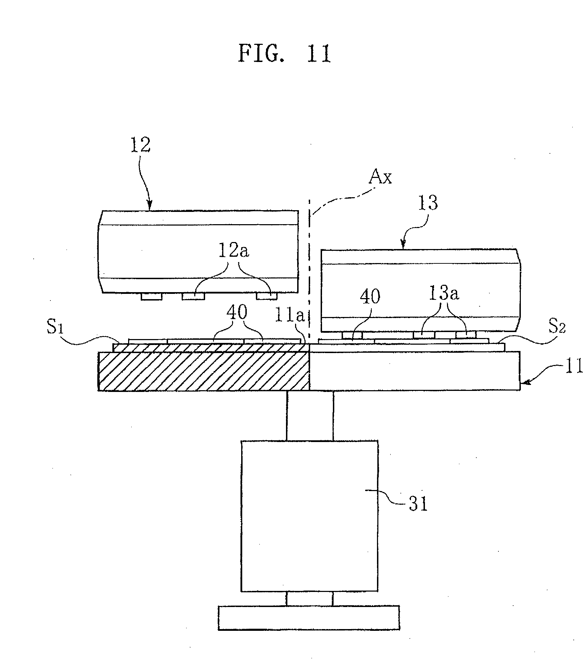

Next, in Step 4, the temperature maintaining step is performed continuously from Step 3 with respect to the first group, whereas the temperature reduction step is performed with respect to the second group, as shown in Fig. 11.

In Step 4, continuously from Step 3, the sample liquid chips 40 of the first group are left in contact with the holding surface 11a and the sample liquid 50 in the sample liquid cell 43 of each of the sample liquid chips 40 is kept at a constant temperature (the lower target temperature T_{L}). Thus, in the sample liquid 50 of the first group, continuously from Step 3, annealing (part of the annealing step of the first group) and elongation (part of the elongation step of the first group) proceed at the same time.

In Step 4, the cooling block 13 is moved closer to the rotation table 11 to come into contact with the sample liquid chips 40 in the second region S₂ of the holding surface 11a by the operation of the driving mechanism 33, as shown in Fig. 11. More specifically, as shown in Fig. 14, each projection 13a of the cooling block 13 is brought into contact with the cell wall 42a of the corresponding sample liquid chip 40. By this contact, the temperature reduction step with respect to the sample liquid chips 40 of the second group is started. In this temperature reduction step, the cell wall 42a of the sample liquid chip 40 is directly cooled by the cooling block 13 or the projection 13a. Heat transfers from the cell wall 42a and the sample liquid 50 in the sample liquid cell 43 to the cooling block 13 or the projection 13a. Thus, the temperature of the sample liquid 50 reduces. During the temperature reduction, annealing gradually proceeds within the sample liquid 50 (part of the annealing process of the second group). The cooling block 13 or the projection 13a is separated from the cell wall 42a of the sample liquid chip 40 by the operation of the driving mechanism 33 immediately before (e.g. 10 to 1000 milliseconds before) the sample liquid 50 reaches the lower target temperature T_{L}. Thus, the heat transfer to the cooling block 13 stops (end of the temperature reduction step of the second group; end of Step 4).

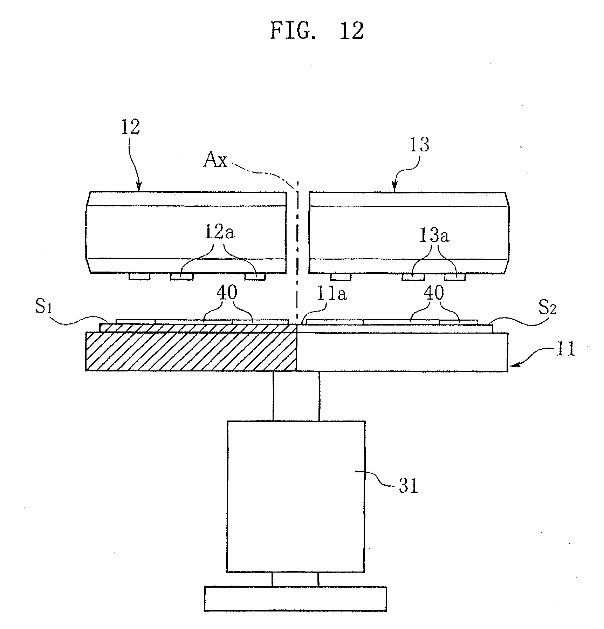

Next, in Step 5, the temperature maintaining step is performed continuously from Step 4 with respect to the first group, and the temperature maintaining step is performed with respect to the second group as well, as shown in Fig. 12.

In Step 5, continuously from Step 4, the sample liquid chips 40 of the first group are left in contact with the holding surface 11a and the sample liquid 50 in the sample liquid cell 43 of each of the sample liquid chips 40 is kept at a constant temperature (the lower target temperature T_{L}). Thus, in the sample liquid 50 of the first group, continuously from Step 4, annealing (part of the annealing step of the first group) and elongation (part of the elongation step of the first group) proceed at the same time.

In Step 5, the sample liquid chips 40 of the second group are left in contact with the holding surface 11a and the sample liquid 50 in the sample liquid cell 43 of each of the sample liquid chips 40 is kept at a constant temperature (the lower target temperature T_{L}) (the temperature maintaining step of the second group). Thus, in the sample liquid 50, annealing (part of the annealing step of the second group) and elongation (part of the elongation step of the second group) proceed at the same time.

Next, in Step 6, the temperature increase step (of the second cycle) is performed with respect to the first group, whereas the temperature maintaining step is performed continuously from Step 5 with respect to the second group, as shown in Fig. 8.

In Step 6, the temperature increase step is performed with respect to the sample liquid chips 40 of the first group in the first region S₁, similarly to the temperature increase step described above with respect to Step 1. Meanwhile, the sample liquid chips 40 of the second group in the second region S₂ are left in contact with the holding surface 11a and the sample liquid 50 in the sample liquid cell 43 of each of the sample liquid chips 40 is kept at a constant temperature (the lower target temperature T_{L}). Thus, in the sample liquid 50 of the second group, annealing (part of the annealing step of the second group) and elongation (part of the elongation step of the second group) proceed at the same time, continuously from Step 5. The temperature maintaining step of the second group is completed when Step 6 is finished.

When Step 6 is finished, the heating block 12 is separated from the sample liquid chips 40 of the first group in the first region S₁, and at the same time, the rotation table 11 is rotated 180° about the axis Ax by the operation of the driving mechanism 31. Due to this rotation, the position of the sample liquid chips 40 in the first region S₁ that belong to the first group switches with the position of the sample liquid chips 40 in the second region S₂ that belong to the second group.

As to Steps 1-6 described above, the temperature increase step of the first group in Step 1 is performed for e.g. six seconds, the temperature reduction step of the first group in Step 2 is performed for e.g. four seconds, and the temperature maintaining step of the first group through Steps 3-5 is performed for e.g. 16 seconds. (The temperature increase step of the first group in Step 6 is performed for the same period of time as that in Step 1.) The temperature increase step of the second group through Steps 2-3 is performed for e.g. six seconds, the temperature reduction step of the second group in Step 4 is performed for e.g. four seconds, and the temperature maintaining step of the second group through Steps 5-6 is performed for e.g. 16 seconds.

In the temperature controlling unit X1, a thermal cycle consisting of the above-described Steps 1-5 is performed repetitively with respect to the sample liquid 50 in the sample liquid cells 43 of the sample liquid chips 40 in the first region S₁ (first group). Thus, the PCR method that repeats a cycle including thermal denaturation, annealing and elongation a predetermined number of times can be performed. In parallel with this, in the temperature controlling unit X1, a thermal cycle consisting of the above-described Steps 2-6 is performed repetitively with respect to the sample liquid 50 in the sample liquid cells 43 of the sample liquid chips 40 in the second region S₂ (second group). Thus, the PCR method that repeats a cycle including thermal denaturation, annealing and elongation a predetermined number of times can be performed also with respect to the sample liquid 50 in the sample liquid cells 43 of the sample liquid chips 40 of the second group. In this way, in the temperature controlling unit X1, the PCR method or the temperature control is performed in a parallel manner.

The temperature controlling unit X1 that operates as described above is suitable for quickly changing the temperature of the sample liquid. The reason is as follows.

The temperature controlling unit X1 is designed such that the heating block 12 can come into direct contact with the cell wall 42a of the sample liquid cell 43 to increase the temperature of the sample liquid 50. Thus, in the temperature increase step, the heating block 12 heats the sample liquid 50 in direct contact with the cell wall 42a. In the above-described conventional PCR machine X2, for example, the heating block 92 needs to heat the tube 94 or the reaction sample liquid in the tube via the holding block 91 (heat capacity member) that holds the tube 94, and the holding block 91 has a large heat capacity. Thus, in the PCR machine X2, to increase the temperature of the reaction sample liquid in the tube 94 to the higher target temperature, it is necessary to increase the temperature of the holding block 91 as well, which has a large heat capacity, to the higher target temperature. Thus, the holding block 91 (heat capacity member) tends to hinder quick temperature increase of the reaction sample liquid. In the temperature increase step by the temperature controlling unit X1, on the other hand, it is not necessary to heat the sample liquid chip 40 or the sample liquid 50 via a heat capacity member for holding the sample liquid chip 40. Thus, the temperature controlling unit X1, in which no member having a large heat capacity intervenes between the heating block 12 and the sample liquid chip 40 or the sample liquid 50, is suitable for quickly increasing the temperature of the sample liquid 50 in the sample liquid cell 43.

With respect to the temperature controlling unit X1, it is supposed that the temperature of the sample liquid 50 in the sample liquid cell 43 during the temperature increase step is represented by T, the amount of heat supplied to the sample liquid 50 is represented by Q, and time is represented by t. Now, the increasing rate of the temperature, i.e., the temperature increase speed of the sample liquid 50 in the temperature increase step (dT/dt) is proportional to the amount of heat supplied to the sample liquid 50 per unit time (dQ/dt). The amount of heat supplied to the sample liquid 50 per unit time (dQ/dt) is highly related to the temperature difference (T₂-T) between the sample liquid 50 and the heating block 12 (kept at a second temperature T₂ higher than the higher target temperature T_{H}) and is substantially proportional to the temperature difference (T₂-T). A larger temperature difference (T₂-T) leads to a larger amount of heat supply to the sample liquid 50 per unit time (dQ/dt) and also to a higher temperature increase speed (dT/dt). In the conventional PCR machine X2, the temperature of the heating block 92 is kept at the thermal denaturation temperature T₁₁ that is the higher target temperature of the reaction sample liquid, and thus the difference from the temperature T of the reaction sample liquid during the temperature increase step is (T₁₁-T). As will be understood by comparing the temperature controlling unit X1 and the conventional PCR machine X2 on the assumption that the higher target temperatures are equal (i.e., T_{H}=T₁₁), the temperature difference (T₂-T) between the sample liquid 50 and the heating block 12 in the temperature controlling unit X1 can be larger than the temperature difference (T₁₁-T) between the reaction sample liquid and the heating block 92 in the PCR machine X2 (T₂>T_{H}=T₁₁). As noted before, a larger temperature difference (T₂-T) leads to a larger amount of heat supply to the sample liquid 50 per unit time (dQ/dt). Accordingly, the temperature increase speed (dT/dt) of the sample liquid 50 is high.

In the temperature increase step with the temperature controlling unit X1, the temperature increase speed (dT/dt) can be made advantageously high in a temperature range close to the higher target temperature T_{H}. As described with reference to Fig. 20, according to the conventional PCR machine X2, the temperature increase speed in a temperature range close to the thermal denaturation temperature T₁₁ (higher target temperature) in the temperature increase step is considerably lowas compared with the temperature increase speed in the initial stage of the temperature increase step. This is because, as the temperature T of the reaction sample liquid increases to approach the thermal denaturation temperature T₁₁ (the higher target temperature), the temperature difference (T₁₁-T) between the reaction sample liquid and the heating block 92 becomes considerably small. (A smaller temperature difference leads to a smaller amount of heat supply to the reaction sample liquid per unit time and hence to a lower temperature increase speed.) On the other hand, according to the temperature controlling unit X1, the temperature difference (T₂-T) between the sample liquid 50 and heating block 12 during temperature increase can be made considerably large even in a temperature range close to the higher target temperature T_{H} in the temperature increase step. Thus, the amount of heat supply to the sample liquid 50 in the sample liquid cell 43 per unit time (dQ/dt) can be made large. Thus, according to the temperature controlling unit X1, the temperature increase speed (dT/dt) in a temperature range close to the higher target temperature T_{H} in the temperature increase step can be made large.

Further, the temperature controlling unit X1 is designed such that the cooling block 13 can come into direct contact with the cell wall 42a of the sample liquid cell 43 to reduce the temperature of the sample liquid 50. Thus, in the temperature reduction step, the coolingblock 13 cools the sample liquid 50 in direct contact with the cell wall 42a. In the above-described conventional PCR machine X2, for example, the cooling block 93 needs cool the tube 94 or the reaction sample liquid in the tube via a holding block 91 (heat capacity member) holding the tube 94, and the holding block 91 has a large heat capacity. Thus, in the PCRmachine X2 , to reduce the temperature of the reaction sample liquid in the tube 94 to the lower target temperature, it is necessary to reduce the temperature of the holding block 91 as well, which has a large heat capacity, to the lower target temperature. Thus, the holding block 91 (heat capacity member) tends to hinder quick temperature reduction of the reaction sample liquid. In the temperature reduction step by the temperature controlling unit X1, on the other hand, the cooling of the sample liquid chip 40 or the sample liquid 50 can be conducted with no intervention of a heat capacity member for holding the sample liquid chip 40. Thus, the temperature controlling unit X1, in which no large heat capacity member intervenes between the cooling block 13 and the sample liquid chip 40 or the sample liquid 50, is suitable for quickly reducing the temperature of the sample liquid 50 in the sample liquid cell 43.

With respect to the temperature controlling unit X1, it is supposed that the temperature of the sample liquid 50 in the sample liquid cell 43 during the temperature reduction step is represented by T, the amount of heat taken from the sample liquid 50 is represented by Q, and time is represented by t. The reducing rate of the temperature, i.e., the temperature reduction speed of the sample liquid 50 in the temperature reduction step (-dT/dt) is proportional to the amount of heat taken from the sample liquid 50 per unit time (dQ/dt). The amount of heat taken from the sample liquid per unit time (dQ/dt) is highly related to the temperature difference (T-T₃) between the sample liquid 50 which is the target to be cooled and the cooling block 13 (kept at a third temperature T₃ lower than the lower target temperature T_{L}) and is substantially proportional to the temperature difference (T-T₃). A larger temperature difference (T-T₃) leads to a larger amount of heat taken from the sample liquid per unit time (dQ/dt) and also to a higher temperature reduction speed (-dT/dt). In the conventional PCR machine X2, the temperature of the cooling block 93 is kept at the annealing/elongation temperature T₁₂ that is the lower target temperature of the reaction sample liquid, and thus the difference from the temperature T of the reaction sample liquid during the temperature reduction step is (T-T₁₂). As will be understood by comparing the temperature controlling unit X1 and the conventional PCR machine X2 on the assumption that the lower target temperatures are equal (i. e. , T_{L}=T₁₂), the temperature difference (T-T₃) between the sample liquid 50 and the cooling block 13 in the temperature controlling uni t X1 can be made larger than the temperature difference (T-T₁₂) between the reaction sample liquid and the cooling block 93 in the PCR machine X2 (T₃<T_{L}=T₁₂). As noted before, a larger temperature difference (T-T₃) leads to a larger amount of heat taken from the sample liquid 50 in the sample liquid cell 43 per unit time (dQ/dt). Thus, the temperature reduction speed (-dT/dt) of the sample liquid 50 is high.

In the temperature controlling unit X1, the temperature reduction speed (-dT/dt) can be made advantageously high in a temperature range close to the lower target temperature T_{L}. As described with reference to Fig. 20, according to the conventional PCR machine X2, the temperature reduction speed in the temperature reduction step in a temperature range close to the annealing/elongation temperature T₁₂ (lower target temperature) is considerably low as compared with the temperature reduction speed in the initial stage of the temperature reduction step. This is because, as the temperature T of the reaction sample liquid reduces to approach the annealing/elongation temperature T₁₂ (the lower target temperature), the temperature difference (T-T₁₂) between the reaction sample liquid and the cooling block 93 becomes considerably small. (A smaller temperature difference leads to a smaller amount of heat taken from the reaction sample liquid per unit time and hence to a lower temperature reduction speed.) On the other hand, according to the temperature controlling unit X1, the temperature difference (T-T₃) between the sample liquid 50 and cooling block 13 during the temperature reduction can be made considerably large even in a temperature range close to the lower target temperature T_{L} in the temperature reduction step. Thus, the amount of heat taken from the sample liquid 50 in the sample liquid cell 43 per unit time (dQ/dt) can be made large. Thus, according to the temperature controlling unit X1, the temperature reduction speed (-dT/dt) in a temperature range close to the lower target temperature T_{L} in the temperature reduction step can be made large.

As described above, the temperature controlling unit X1 is suitable for quickly changing (increasing or reducing) the temperature of the sample liquid 50. Although the temperature controlling unit X1 is suitable for use as a PCR machine, which requires quick temperature control, the temperature controlling unit can be used also as other kinds of temperature controlling unit.

Moreover, the temperature controlling unit X1 is suitable for controlling the temperature of the sample liquid 50 precisely to the higher target temperature T_{H} or the lower target temperature T_{L}. The reason is as follows.

In theory, in the conventional PCR machine X2, as the temperature of the reaction sample liquid T approaches the thermal denaturation temperature T₁₁, the reaction sample liquid temperature T and the temperature T₁₁ of the heating block 92 become closer to each other, and the temperature increase speed (dT/dt) of the reaction sample liquid, which is substantially proportional to the temperature difference (T₁₁-T), approaches 0. Thus, in theory, in the conventional PCR machine X2, the reaction sample liquid temperature T cannot reach the thermal denaturation temperature T₁₁ (higher target temperature) within a finite time in the temperature increase step. In practice as well, in the conventional PCR machine X2, the reaction sample liquid temperature T hardly reaches the thermal denaturation temperature T₁₁ in the temperature increase step. Thus, it is difficult to cause the reaction sample liquid temperature T to reach the precise thermal denaturation temperature T₁₁. In contrast, in the temperature controlling unit X1, the temperature increase speed (dT/dt) of the sample liquid 50 can be made high in a temperature range close to the higher target temperature T_{H} in the temperature increase step, as described above. This means that the temperature increase speed (dT/dt) of the sample liquid 50 can be kept high until the temperature T of the sample liquid 50 reaches the higher target temperature T_{H} so that the temperature T of the sample liquid 50 reaches the higher target temperature T_{H} quickly and reliably. By separating the heating block 12 from the sample liquid chip 40 when the temperature T of the sample liquid 50 in the sample liquid chip 40 has reached the higher target temperature T_{H}, heat transfer from the heating block 12 to the sample liquid chip 40 or the sample liquid 50 can be stopped, whereby temperature increase of the sample liquid 50 can be stopped. The temperature controlling unit X1 having such a structure is suitable for controlling the temperature T of the sample liquid 50 precisely to the higher target temperature T_{H}.

Generally, in reducing the temperature of a liquid by continuously taking heat from the liquid, the temperature of the liquid sometimes continues to drop even after the taking of heat from the liquid is stopped. For instance, in the above-described temperature reduction step of the conventional PCR machine X2, the cooling block 93 is separated from the holding block 91 to stop taking heat from the reaction sample liquid when the reaction sample liquid temperature T reaches the annealing/elongation temperature T₁₂ (the lower target temperature). However, even after this, the reaction sample liquid temperature sometimes continues to drop below the annealing/elongation temperature T₁₂. Thus, with the conventional PCR machine X2, it is difficult to control the reaction sample liquid temperature T precisely to the annealing/elongation temperature T₁₂. In the temperature controlling unit X1, the rotation table 11 holds the sample liquid chip 40 in contact with the cell wall 41a of the sample liquid cell 43 of the sample liquid chip 40. (The temperature of the rotation table 11 is set to and kept at the first temperature T₁ for keeping the sample liquid 50 at the lower target temperature T_{L}). This prevents the temperature T of the sample liquid 50 from continuing to drop after the separation of the cooling block 13 from the cell wall 42a. The temperature controlling unit X1 having this arrangement is suitable for controlling the temperature T of the sample liquid 50 in the temperature reduction step precisely to the lower target temperature T_{L}.

As described above, the temperature controlling unit X1 is suitable for controlling the sample liquid 50 precisely to the higher target temperature T_{H} or the lower target temperature T_{L}. Although this temperature controlling unit X1 is suitable for use as a PCR machine, which requires precise temperature control, the temperature controlling unit can be used also as other kinds of temperature controlling unit.

As noted before, in the temperature controlling unit X1, the heating block 12 and the cooling block 13 can be individually brought into contact with a side of the sample liquid chip 40 (i.e., the cell wall 42a) that is opposite from the rotation table 11. This arrangement is suitable for efficiently realizing holding of the sample liquid chips 40 on the rotation table 11, which is kept at a constant temperature, movement of the heating block 12 for coming into contact with the sample liquid chips 40 (operation for temperature increase of the sample liquid 50) and movement of the cooling block 13 for coming into contact with the sample liquid chips 40 (operation for temperature reduction of the sample liquid 50).

As noted before, in the temperature controlling unit X1, the rotation table 11 has a holding surface 11a for holding the sample liquid chips 40 and is rotatable around the axis Ax perpendicular to the holding surface 11a. Further, each of the heating block 12 and the cooling block 13 faces the holding surface 11a of the rotation table 11 and is movable toward and away from the holding surface 11a. This arrangement is suitable for efficiently realizing holding of the sample liquid chips 40 on the rotation table 11, which is kept at a constant temperature, movement of the heating block 12 for coming into contact with the sample liquid chips 40 (operation for temperature increase of the sample liquid 50) and movement of the cooling block 13 for coming into contact with the sample liquid chips 40 (operation for temperature reduction of the sample liquid 50).

As described above, in the temperature controlling unit X1, the holding surface 11a includes the first region S₁ configured to hold a plurality of sample liquid chips 40 in contact with the sample liquid chips, and the second region S2 configured to hold a plurality of sample liquid chips 40 in contact with the sample liquid chips. Moreover, each of the heating block 12 and the cooling block 13 is configured to come into contact with a plurality of sample liquid chips 40 held in the first region S₁ when the heating block or the cooling block faces the first region S₁, and configured to come into contact with a plurality of sample liquid chips 40 held in the second region S₂ when the heating block or the cooling block faces the second region S₂. With this arrangement, it is possible to perform in parallel the temperature increase step with respect to the sample liquid 50 in the sample liquid chips 40 in the first region S₁ by the heating block 12 and the temperature reduction step with respect to the sample liquid 50 in the sample liquid chips 40 in the second region S₂ by the cooling block 13. Further, it is possible to perform in parallel the temperature increase step with respect to the sample liquid 50 in the sample liquid chips 40 in the second region S₂ by the heating block 12 and the temperature reduction step with respect to the sample liquid 50 in the sample liquid chips 40 in the first region S₁ by the cooling block 13.

As described above, in the temperature controlling unit X1, the first region S₁ and the second region S₂ are configured to hold a plurality of sample liquid chips 40 such that the sample liquid chips 40 are arranged on a circle (imaginary circle) around the axis Ax. This arrangement allows switching the position of the sample liquid chips 40 held in the first region S₁ and the position of the sample liquid chips 40 held in the second region S₂ by 180° rotation of the rotation table 11 or the holding surface 11a (rotation about the axis Ax).

As described above, in the temperature controlling unit X1, the sample liquid chip 40 includes cell walls 41a and 42a facing and spaced from each other, and a sample liquid cell 43 for receiving sample liquid 50 is defined between the cell walls 41a and 42a. Further, the rotation table 11 can hold the sample liquid chip 40 in contact with the cell wall 41a of the sample liquid chip 40, the heating block 12 can come into contact with the cell wall 42a of the sample liquid chip 40, and the cooling block 13 can also come into contact with the cell wall 42a of the sample liquid chip 40. This arrangement is suitable for efficient heat transfer between sample liquid 50 as a temperature control target and the heating block 12, and heat transfer between the sample liquid 50 and the cooling block 13.

As described above, in the temperature controlling unit X1, the maximum dimension of the sample liquid cell 43 in a direction perpendicular to the spacing direction (vertical direction in Fig. 5B) of the cell walls 41a and 42a is larger than the maximum dimension of the sample liquid cell 43 in the spacing direction. That is, the sample liquid cell 43 for receiving the sample liquid as a temperature control target is shallow. This arrangement is suitable for increasing the surface area of the sample liquid 50 per unit volume. A large surface area per unit volume of the sample liquid 50 contributes to efficient heat transfer between the sample liquid 50 and the heating block 12 and the heat transfer between the sample liquid 50 and the cooling block 13.

As described above, in the temperature controlling unit X1, the heating block 12 and the cooling block 13 include projections 12a and projections 13a, respectively, for coming into contact with the cell walls 42. This arrangement is suitable for allowing local heat transfer from the heating block 12 to the sample liquid 50 in the sample liquid cell 43 and local heat transfer from the sample liquid 50 in the sample liquid cell 43 to the cooling block 13. Realizing local heat transfer contributes to enhancement of heat transfer efficiency.

### EXAMPLE

The above-described temperature controlling unit X₁ was used, and temperature change of a liquid as a temperature control target was measured. Specifically, these were performed as follows.

First, a sample liquid chip 40 with a thermocouple inserted in the sample liquid cell 43 was prepared, and the sample liquid chip 40 was set in the first region S₁ of the holding surface 11a of the rotation table 11. Then, sample liquid 50 was introduced into the sample liquid cell 43 and mineral oil 60 was supplied into the liquid retaining space 44 in the same manner as shown in Figs. 6A-6C. The thermocouple of the sample liquidchip 40 was arranged to constantlymeasure the temperature of the sample liquid 50 in the sample liquid cell 43 by utilizing a circuit provided at the rotation table 11. By operating the rotation table 11, the heating block 12 and the cooling block 13 of the temperature controlling unit X1, the thermal cycle consisting of the temperature increase step of Step 1, the temperature reduction step of Step 2 and the temperature maintaining step of Step 3-5 was repetitively performed with respect to the sample liquid 50 in the sample liquid cell 43 of the sample liquid chip 40, in the same manner as described above with respect to the sample liquid 50 in the sample liquid cells 43 of the sample liquid chips 40 of the first group.

In this Example, the room temperature was 25 °C, and the higher target temperature T_{H} and the lower target temperature T_{L} for the sample liquid 50 were set to 95 °C and 62 °C, respectively. The temperature of the holding surface 11a of the rotation table 11 (first temperature T₁) was set to 73 °C, the temperature of the heating block 12 (second temperature T₂) was set to 120 °C, and the temperature of the cooling block 13 (third temperature T₃) was set to 40 °C. The temperature increase step was performed for six seconds, the temperature reduction step was performed for four seconds, and the temperature maintaining step was performed more than 16 seconds. In the temperature increase step of this Example, the heating block 12 was separated from the cell wall 42a of the sample liquid chip 40 when the temperature of the sample liquid 50 reached the higher target temperature T_{H}. In the temperature reduction step of this Example, the cooling block 13 was separated from the cell wall 42a of the sample liquid chip 40 one hundred milliseconds before the time when the temperature of the sample liquid 50 was expected to reach the lower target temperature T_{L} (the expected time determined in advance based on experiments or the like).

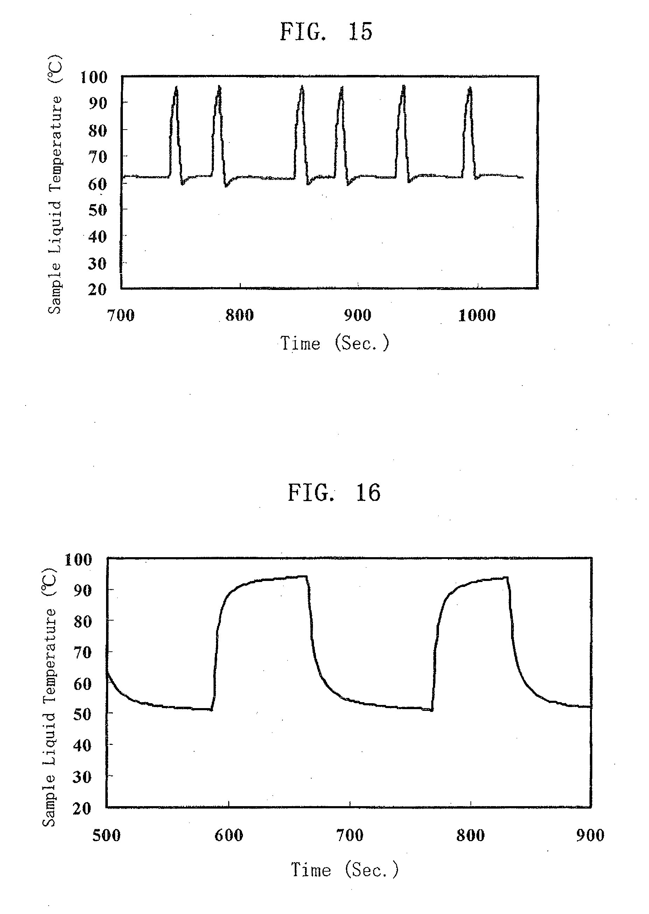

Part of the temperature change measured in this Example is shown in the graph of Fig. 15. In the graph of Fig. 15, the horizontal axis indicates time (seconds), whereas the vertical axis indicates sample liquid temperature (°C). It is clear from the temperature change shown in the graph of Fig. 15 that the temperature controlling unit X1 can change the temperature of the sample liquid 50 (liquid) quickly and precisely.

### COMPARATIVE EXAMPLE

The temperature controlling unit X1, with the temperature control function of the rotation table 11 stopped, was used, and temperature change of a liquid as a temperature control target was measured. Specifically, these were performed as follows.

Similarly to the above-described Example, a sample liquid chip 40 with a thermocouple (and with the sample liquid cell 43 containing sample liquid 50) was prepared and set in the first region S₁ of the holding surface 11a. Similarly to the Example, the thermocouple of the sample liquid chip 40 was arranged to constantly measure the temperature of the sample liquid 50 in the sample liquid cell 43. In this Comparative Example, the room temperature was 25 °C, and the higher target temperature T_{H} and the lower target temperature T_{L} for the sample
liquid 50 were set to 95 °C and 50 °C, respectively. In this Comparative Example, the temperature of the heating block 12 (second temperature T₂) was set to 100 °C, and the temperature of the cooling block 13 (third temperature T₃) was set to 50 °C. By operating the rotation table 11 (the temperature control function stopped), the heating block 12 and the cooling block 13 of the temperature controlling unit X1, the thermal cycle consisting of a predetermined temperature increase step and a predetermined temperature reduction step was repetitively performed with respect to the sample liquid 50 in the sample liquid cell 43 of the sample liquid chip 40. In the temperature increase step, the heating block 12 was separated from the cell wall 42a of the sample liquid chip 40 when the temperature of the sample liquid 50 reached 95 °C, which was the higher target temperature T_{H}. In the temperature reduction step, the cooling block 13 was separated from the cell wall 42a of the sample liquid chip 40 when the temperature of the sample liquid 50 reached 50 °C, which was the lower target temperature T_{L}.

The temperature change measured in this Comparative Example is shown in the graph of Fig. 16. In the graph of Fig. 16, the horizontal axis indicates time (second), whereas the vertical axis indicates sample liquid temperature (°C). It is clear from the temperature change shown in the graph of Fig. 16 that it is difficult to change the temperature of the sample liquid 50 (liquid) quickly and precisely according to the Comparative Example. In the temperature increase step of the Comparative Example, it took about 80 seconds to raise the temperature of the sample liquid 50 from about 50 °C to about 95 °C. In the temperature reduction step of this Comparative Example, it took about 95 seconds to reduce the temperature of the sample liquid from about 95 °C to about 50 °C.

## Claims

1. A temperature controlling unit (X1) comprising:
a holder (11) for holding a liquid receiver (40) containing a liquid (50) in contact with the liquid receiver, the holder being configured to maintain a first temperature (T₁) for keeping temperature of the liquid at a lower target temperature (T_{L});
a heating block (12) for increasing the temperature of the liquid through contact with the liquid receiver, the heating block being movable relative to the liquid receiver and configured to maintain a second temperature (T₂) higher than a higher target temperature (T_{H}) that is higher than the lower target temperature (T_{L}); and
a cooling block (13) for reducing the temperature of the liquid through contact with the liquid receiver, the cooling block being movable relative to the liquid receiver and configured to maintain a third temperature (T₃) lower than the lower target temperature (T_{L});
wherein: the holder includes a holding surface (11a) for holding the liquid receiver and is rotatable about an axis (Ax) perpendicular to the holding surface; and
each of the heating block and the cooling block faces the holding surface of the holder and is movable toward and away from the holding surface.

2. The temperature controlling unit according to claim 1, wherein the first temperature is any one of a temperature equal to the lower target temperature, a temperature higher than the lower target temperature and lower than the higher target temperature, and a temperature lower than the lower target temperature and higher than the third temperature.

3. The temperature controlling unit according to claim 1, wherein: the heating block is configured to come into contact with a side (42a) of the liquid receiver that is opposite from the holder, and
the cooling block is configured to come into contact with a side (42a) of the liquid receiver that is opposite from the holder.

4. The temperature controlling unit according to claim 1, wherein: the holding surface includes a first region (S₁) for holding a liquid receiver (40) containing a liquid (50) in contact with the liquid receiver and a second region (S₂) for holding a liquid receiver (40) containing a liquid (50) in contact with the liquid receiver; and
each of the heating block and the cooling block is configured to move closer to and come into contact with the liquid receiver held in the first region when facing the first region and configured to move closer to and come into contact with the liquid receiver held in the second region when facing the second region.

5. The temperature controlling unit according to claim 1, wherein: the holding surface includes a first region (S₁) for holding a plurality of liquid receivers (40) each containing a liquid (50) in contact with the liquid receivers and a second region (S₂) for holding a plurality of liquid receivers (40) each containing a liquid (50) in contact with the liquid receivers; and
each of the heating block and the cooling block is configured to move closer to and come into contact with the plurality of liquid receivers held in the first region when facing the first region and configured to move closer to and come into contact with the plurality of liquid receivers held in the second region when facing the second region.

6. The temperature controlling unit according to claim 5 , wherein the first region and the second region are configured to hold the plurality of liquid receivers such that the liquid receivers are arranged on a circle around the axis.

7. The temperature controlling unit according to claim 1, wherein: the liquid receiver includes a first cell wall (41 a) and a second cell wall (42a) facing and spaced from each other, and a cell (43) for receiving a liquid defined between the first cell wall and the second cell wall;
the holder is configured to hold the liquid receiver in contact with the first cell wall of the liquid receiver;
the heating block is configured to come into contact with the second cell wall of the liquid receiver; and
the cooling block is configured to come into contact with the second cell wall of the liquid receiver.

8. The temperature controlling unit according to claim 7 , wherein a maximum dimension of the cell in a direction perpendicular to a spacing direction in which the first cell and the second cell are spaced from each other is larger than a maximum dimension of the cell in the spacing direction.

9. The temperature controlling unit according to claim 7, wherein each of the heating block and the cooling block includes a projection (12a, 13a) for coming into contact with the second cell wall.

10. A temperature controlling method for controlling the temperature of a liquid by using the controlling unit of claim 1, the method comprising:
providing a liquid (50) in the liquid receiver (40) held in the holder (11a);
a temperature increase step (step 1; step 6) for bringing the heating block (12) kept at a heating temperature (T₂) higher than a higher target temperature (T_{H}) for the liquid into contact with the liquid receiver containing the liquid to increase the temperature of the liquid; and
a temperature reduction step (step 2; step 4) for bringing the cooling block (13) kept at a cooling temperature (T₃) lower than a lower target temperature (T_{L}) that is lower than the higher target temperature (T_{H}) into contact with the liquid receiver to reduce the temperature of the liquid;
wherein the holder (11a) is maintained at the first temperature (T₁) for keeping a temperature of the liquid at the lower target temperature (T_{L}), said first temperature (T₁) being any one of a temperature equal to the lower target temperature (T_{L}), a temperature higher than the lower target temperature (T_{L}) and lower than the higher target temperature (T_{H}), and a temperature lower than the lower target temperature (T_{L}) and higher than the cooling temperature (T₃); and
each of the heating block and the cooling block is movable toward and away from the holder (11a).

11. The temperature controlling method according to claim 10, wherein, in the temperature increase step, the heating block is separated from the liquid receiver when the temperature of the liquid has reached the higher target temperature.

12. The temperature controlling method according to claim 10, wherein, in the temperature reduction step, the cooling block is separated from the liquid receiver before the temperature of the liquid reaches the lower target temperature.

## Patentansprüche

1. Eine Temperatursteuerungseinheit (X1) aufweisend:
einen Halter (11) zum Halten eines Flüssigkeitssammlers (40), der eine Flüssigkeit (50) enthält, die den Flüssigkeitssammler kontaktiert, wobei der Halter dazu konfiguriert ist, um eine erste Temperatur (T₁) für ein Halten einer Temperatur von der Flüssigkeit auf einer niedrigeren Zieltemperatur (T_{L}) beizubehalten;
einen Heizblock (12) zum Erhöhen der Temperatur von der Flüssigkeit durch Kontakt mit dem Flüssigkeitssammler, wobei der Heizblock relativ zu dem Flüssigkeitssammler beweglich und dazu konfiguriert ist, um eine zweite Temperatur (T₂), die höher als eine höhere Zieltemperatur (T_{H}) ist, die höher als die niedrigere Zieltemperatur (T_{L}) ist, beizubehalten; und
einen Kühlblock (13) zum Reduzieren der Temperatur von der Flüssigkeit durch Kontakt mit dem Flüssigkeitssammler, wobei der Kühlblock relativ zu dem Flüssigkeitssammler beweglich und dazu konfiguriert ist, um eine dritten Temperatur (T₃), die niedriger als die niedrigere Zieltemperatur (T_{L}) ist, beizubehalten, wobei:
der Halter eine Haltefläche (11 a) zum Halten des Flüssigkeitssammlers umfasst und um eine Achse (Ax) senkrecht zu der Haltefläche drehbar ist; und
jeder von dem Heizblock und dem Kühlblock der Haltefläche von dem Halter gegenüberliegt und in Richtung und weg von der Haltefläche beweglich ist.

2. Die Temperatursteuerungseinheit nach Anspruch 1, wobei die erste Temperatur eine von einer Temperatur, die gleich der niedrigeren Temperatur ist, einer Temperatur, die höher als die niedrigere Zieltemperatur und niedriger als die höhere Zieltemperatur ist und einer Temperatur, die niedriger als die niedrigere Zieltemperatur und höher als die dritte Temperatur ist, ist.

3. Die Temperatursteuerungseinheit nach Anspruch 1, wobei:
der Heizblock dazu konfiguriert ist, um in Kontakt mit einer Seite (42a) von dem Flüssigkeitssammler zu kommen, die dem Halter gegenüberliegt, und
der Kühlblock dazu konfiguriert ist, um in Kontakt mit einer Seite (42a) von dem Flüssigkeitssammler zu kommen, die dem Halter gegenüberliegt.

4. Die Temperatursteuerungseinheit nach Anspruch 1, wobei:
die Halterfläche einen ersten Bereich (S₁) zum Halten eines Flüssigkeitssammlers (40), der eine Flüssigkeit (50) enthält, die den Flüssigkeitssammler kontaktiert, und einen zweiten Bereich (S₂) zum Halten eines Flüssigkeitssammlers (40), der eine Flüssigkeit (50) enthält, die den Flüssigkeitssammler kontaktiert, umfasst; und
wobei jeder von dem Heizblock und dem Kühlblock dazu konfiguriert ist, um sich näher an den Flüssigkeitssammler heran zu bewegen und den Flüssigkeitssammler zu kontaktieren, der in dem ersten Bereich gehalten ist, wenn dem ersten Bereich gegenüberliegend, und dazu konfiguriert ist, um sich näher an den Flüssigkeitssammler heran zu bewegen und den Flüssigkeitssammler zu kontaktieren, der in dem zweiten Bereich gehalten ist, wenn dem zweiten Bereich gegenüberliegend.

5. Die Temperatursteuerungseinheit nach Anspruch 1, wobei:
die Haltefläche einen ersten Bereich (S₁) zum Halten einer Vielzahl von Flüssigkeitssammlern (40), wovon jeder eine Flüssigkeit (50) enthält, die die Flüssigkeitssammler kontaktiert, und einen zweiten Bereich (S₂) zum Halten einer Vielzahl von Flüssigkeitssammlern (40), wovon jeder eine Flüssigkeit (50) enthält, die die Flüssigkeitssammler kontaktiert, umfasst; und
wobei jeder von dem Heizblock und dem Kühlblock dazu konfiguriert sind, um sich näher an die Vielzahl von Flüssigkeitssammlern heran zu bewegen und mit der Vielzahl von Flüssigkeitssammlern in Kontakt zu kommen, die in dem ersten Bereich gehalten sind, wenn dem ersten Bereich gegenüberliegend, und dazu konfiguriert sind, um sich näher an die Vielzahl von Flüssigkeitssammlern heran zu bewegen und mit der Vielzahl von Flüssigkeitssammlern in Kontakt zu kommen, die in dem zweiten Bereich gehalten sind, wenn dem zweiten Bereich gegenüberliegend.

6. Die Temperatursteuerungseinheit nach Anspruch 5, wobei der erste Bereich und der zweite Bereich dazu konfiguriert sind, um die Vielzahl von Flüssigkeitssammlern zu halten, so dass die Flüssigkeitssammler auf einem Kreis um die Achse herum angeordnet sind.

7. Die Temperatursteuerungseinheit nach Anspruch 1, wobei:
der Flüssigkeitssammler eine erste Zellenwand (41 a) und eine zweite Zellenwand (42a), die einander zugewandt und voneinander beabstandet sind, und eine Zelle (43) zum Empfangen einer Flüssigkeit umfasst, die zwischen der ersten Zellenwand und der zweiten Zellenwand definiert ist;
der Halter dazu konfiguriert ist, um den Flüssigkeitssammler in Kontakt mit der ersten Zellenwand von dem Flüssigkeitssammler zu halten;
der Heizblock dazu konfiguriert ist, um mit der zweiten Zellenwand von dem Flüssigkeitssammler in Kontakt zu kommen; und
der Kühlblock dazu konfiguriert ist, um mit der zweiten Zellenwand von dem Flüssigkeitssammler in Kontakt zu kommen.

8. Die Temperatursteuerungseinheit nach Anspruch 7, wobei eine maximale Abmessung von der Zelle in einer Richtung senkrecht zu einer Beabstandungsrichtung, in der die erste Zelle und die zweite Zelle voneinander beabstandet sind, größer als eine maximale Abmessung von der Zelle in der Beabstandungsrichtung ist.

9. Die Temperatursteuerungseinheit nach Anspruch 7, wobei jeder von dem Heizblock und dem Kühlblock einen Vorsprung (12a, 13a) umfasst, um mit der zweiten Zellenwand in Kontakt zu kommen.

10. Ein Temperatursteuerungsverfahren zum Steuern der Temperatur von einer Flüssigkeit durch Verwenden der Steuerungseinheit nach Anspruch 1, das Verfahren umfassend:
Bereitstellen einer Flüssigkeit (50) in dem Flüssigkeitssammler (40), der in dem Halter (11a) gehalten ist;
einen Temperaturerhöhungsschritt (Schritt 1; Schritt 6), um den Heizblock (12), der auf einer Heiztemperatur (T₂), die höher als eine höhere Zieltemperatur (T_{H}) für die Flüssigkeit ist, gehalten ist, in Kontakt mit dem Flüssigkeitssammler, der die Flüssigkeit enthält, zu bringen, um die Temperatur von der Flüssigkeit zu erhöhen; und
einen Temperaturreduzierungsschritt (Schritt 2; Schritt 4), um den Kühlblock (13), der auf einer Kühltemperatur (T₃), die niedriger als eine niedrigere Zieltemperatur (T_{L}) ist, die niedriger als die höhere Zieltemperatur (T_{H}) ist, gehalten ist, in Kontakt mit dem Flüssigkeitssammler zu bringen, um die Temperatur von der Flüssigkeit zu reduzieren;
wobei der Halter (11a) auf der ersten Temperatur (T₁) zum Halten einer Temperatur von der Flüssigkeit auf der niedrigeren Zieltemperatur (T_{L}) beibehalten wird, wobei die besagte erste Temperatur (T₁) eine von einer Temperatur, die gleich der niedrigeren Zieltemperatur (T_{L}) ist, einer Temperatur, die höher als die niedrigere Zieltemperatur (T_{L}) ist und niedriger als die höhere Zieltemperatur (T_{H}) ist, und einer Temperatur, die niedriger als die niedrigere Zieltemperatur (T_{L}) und höher als die Kühltemperatur (T₃) ist, ist; und
jeder von dem Heizblock und dem Kühlblock in Richtung und weg von dem Halter (11a) bewegbar ist.

11. Das Temperatursteuerungsverfahren nach Anspruch 10, wobei in dem Temperaturerhöhungsschritt der Heizblock von dem Flüssigkeitssammler getrennt wird, wenn die Temperatur von der Flüssigkeit die höhere Zieltemperatur erreicht hat.

12. Das Temperatursteuerungsverfahren nach Anspruch 10, wobei in dem Temperaturreduzierungsschritt der Kühlblock von dem Flüssigkeitssammler getrennt wird, bevor die Temperatur von der Flüssigkeit die niedrigere Zieltemperatur erreicht.

## Revendications

1. Unité de commande de température (X1) comprenant :
un support (11) pour supporter un récepteur de liquide (40) contenant un liquide (50) en contact avec le récepteur de liquide, le support étant configuré pour maintenir une première température (T₁) pour conserver la température du liquide à une température cible inférieure (T_{L}) ;
un bloc de chauffage (12) pour augmenter la température du liquide par contact avec le récepteur de liquide, le bloc de chauffage étant mobile par rapport au récepteur de liquide et configuré pour maintenir une deuxième température (T₂) plus élevée qu'une température cible supérieure (T_{H}) qui est plus haute que la température cible inférieure (T_{L}) ; et
un bloc de refroidissement (13) pour réduire la température du liquide par contact avec le récepteur de liquide, le bloc de refroidissement étant mobile par rapport au récepteur de liquide et configuré pour maintenir une troisième température (T₃) plus basse que la température cible inférieure (T_{L}) ;
dans laquelle : le support inclut une surface de support (11a) pour supporter le récepteur de liquide et est rotatif autour d'un axe (Ax) perpendiculaire à la surface de support ; et
chacun du bloc de chauffage et du bloc de refroidissement fait face à la surface de support du support et est mobile vers et loin de la surface de support.

2. Unité de commande de température selon la revendication 1, dans laquelle la première température est l'une quelconque d'une température égale à la température cible inférieure, d'une température plus élevée que la température cible inférieure et plus basse que la température cible supérieure, et d'une température plus basse que la température cible inférieure et plus haute que la troisième température.

3. Unité de commande de température selon la revendication 1, dans laquelle : le bloc de chauffage est configuré pour entrer en contact avec un côté (42a) du récepteur de liquide qui est opposé au support, et
le bloc de refroidissement est configuré pour entrer en contact avec un côté (42a) du récepteur de liquide qui est opposé au support.

4. Unité de commande de température selon la revendication 1, dans laquelle : la surface de support inclut une première région (S₁) pour supporter un récepteur de liquide (40) contenant un liquide (50) en contact avec le récepteur de liquide et une seconde région (S₂) pour supporter un récepteur de liquide (40) contenant un liquide (50) en contact avec le récepteur de liquide ; et
chacun du bloc de chauffage et du bloc de refroidissement est configuré pour se déplacer tout près et entrer en contact avec le récepteur de liquide supporté dans la première région en faisant face à la première région et configuré pour se déplacer tout près et entrer en contact avec le récepteur de liquide supporté dans la seconde région en faisant face à la seconde région.

5. Unité de commande de température selon la revendication 1, dans laquelle : la surface de support inclut une première région (S₁) pour supporter une pluralité de récepteurs de liquide (40) chacun contenant un liquide (50) en contact avec les récepteurs de liquide et une seconde région (S₂) pour supporter une pluralité de récepteurs de liquide (40) chacun contenant un liquide (50) en contact avec les récepteurs de liquide ; et
chacun du bloc de chauffage et du bloc de refroidissement est configuré pour se déplacer tout près et entrer en contact avec la pluralité de récepteurs de liquide supportés dans la première région en faisant face à la première région et configuré pour se déplacer tout près et entrer en contact avec la pluralité de récepteurs de liquide supportés dans la seconde région en faisant face à la seconde région.

6. Unité de commande de température selon la revendication 5, dans laquelle la première région et la seconde région sont configurées pour supporter la pluralité de récepteurs de liquide de sorte que les récepteurs de liquide sont agencés sur un cercle autour de l'axe.

7. Unité de commande de température selon la revendication 1, dans laquelle : le récepteur de liquide inclut une première paroi cellulaire (41a) et une seconde paroi cellulaire (42a) se faisant face et espacées l'une de l'autre, et une cellule (43) pour recevoir un liquide définie entre la première paroi cellulaire et la seconde paroi cellulaire ;
le support est configuré pour supporter le récepteur de liquide en contact avec la première paroi cellulaire du récepteur de liquide ;
le bloc de chauffage est configuré pour entrer en contact avec la seconde paroi cellulaire du récepteur de liquide ; et
le bloc de refroidissement est configuré pour entrer en contact avec la seconde paroi cellulaire du récepteur de liquide.

8. Unité de commande de température selon la revendication 7, dans laquelle une dimension maximale de la cellule dans une direction perpendiculaire à une direction d'espacement dans laquelle la première cellule et la seconde cellule sont espacées l'une de l'autre est plus grande qu'une dimension maximale de la cellule dans la direction d'espacement.

9. Unité de commande de température selon la revendication 7, dans laquelle chacun du bloc de chauffage et du bloc de refroidissement inclut une partie en saillie (12a, 13a) pour entrer en contact avec la seconde paroi cellulaire.

10. Procédé de commande de température pour commander la température d'un liquide en utilisant l'unité de commande de la revendication 1, le procédé comprenant :
la fourniture d'un liquide (50) dans le récepteur de liquide (40) supporté dans le support (11 a) ;
une étape d'augmentation de température (étape 1 ; étape 6) pour amener le bloc de chauffage (12) conservé à une température de chauffage (T₂) plus haut qu'une température cible supérieure (T_{H}) pour le liquide en contact avec le récepteur de liquide contenant le liquide pour augmenter la température du liquide ; et
une étape de réduction de température (étape 2 ; étape 4) pour amener le bloc de refroidissement (13) conservé à une température de refroidissement (T₃) plus basse qu'une température cible inférieure (T_{L}) qui est plus basse que la température cible supérieure (TH) en contact avec le récepteur de liquide pour réduire la température du liquide ;
dans lequel le support (11a) est maintenu à la première température (T₁) pour conserver une température du liquide à la température cible inférieure (T_{L}), ladite première température (T₁) étant l'une quelconque d'une température égale à la température cible inférieure (T_{L}), d'une température plus élevée que la température cible inférieure (T_{L}) et plus basse que la température cible supérieure (T_{H}), et d'une température plus basse que la température cible inférieure (T_{L}) et plus élevée que la température de refroidissement (T₃) ; et
chacun du bloc de chauffage et du bloc de refroidissement est mobile vers et loin du support (11a).

11. Procédé de commande de température selon la revendication 10, dans lequel, dans l'étape d'augmentation de température, le bloc de chauffage est séparé du récepteur de liquide quand la température du liquide a atteint la température cible supérieure.

12. Procédé de commande de température selon la revendication 10, dans lequel, dans l'étape de réduction de température, le bloc de refroidissement est séparé du récepteur de liquide avant que la température du liquide n'atteigne la température cible inférieure.
